# EUROPÄISCHE PATENTSCHRIFT

(11) **EP 1 740 657 B1**
(45) Veröffentlichungstag und Bekanntmachung des Hinweises auf die Patenterteilung: **12.09.2007**
(21) Anmeldenummer: 04803818.6
(22) Anmeldetag: 13.12.2004
(51) Int. Cl.: C09B 31/02

(54) **KATIONISCHE NAPHTHYLDIAZOFARBSTOFFE SOWIE DIESE FARBSTOFFE ENTHALTENDE MITTEL ZUR FÄRBUNG VON KERATINFASERN**
CATIONIC NAPHTHYLDIAZO DYES AND AGENTS CONTAINING SAID DYES USED TO COLOUR KERATIN FIBRES
COLORANTS NAPHTYLDIAZO CATIONIQUES ET AGENTS CONTENANT CES COLORANTS DESTINES A LA COLORATION DE FIBRES KERATINIQUES

(30) Priorität: 06.03.2004 DE 102004010999
(43) Veröffentlichungstag der Anmeldung: 10.01.2007
(73) Patentinhaber: Wella Aktiengesellschaft, 64274 Darmstadt (DE)
(72) Erfinder: GÖTTEL, Otto, CH-1723 Marly (CH); HAYOZ, André, CH-1724 Senèdes (CH); BRAUN, Hans-Jürgen, CH-3182 Ueberstorf (CH)
(74) Vertreter: Bockhorst, Matthias
(86) Internationale Anmeldenummer: PCT/EP2004/014189
(87) Internationale Veröffentlichungsnummer: WO 2005/085362

(56) Entgegenhaltungen:
- EP-A- 0 159 549
- WO-A-00/42980
- WO-A-03/022233
- GB-A- 1 299 080

## Beschreibung

Die vorliegende Erfindung betrifft neue direktziehende Farbstoffe zur Herstellung von nicht-oxidativen Färbemitteln für Keratinfasern, wie zum Beispiel Haare, Seide oder Wolle.

Die Färbung von Haaren ist in der heutigen Zeit den unterschiedlichsten Trends unterworfen. Während früher Haare vor allem gefärbt wurden, um Grauanteile langandauernd zu überdecken, besteht heute immer mehr das Verlangen nach der Integration der Haarfarbe in die aktuelle Mode sowie als Ausdruck der Persönlichkeit.

Zur Färbung von Haaren stehen nach wie vor zwei Methoden zur Verfügung. Einerseits ist dies die oxidative Haarfärbung, die zu sehr dauerhaften Färbeergebnissen führt; andererseits sind dies nicht-oxidative Tönungen, die in der Regel schonender als oxidative Färbungen aber auch weniger haltbar sind. Derartige nicht-oxidative Färbemittel sind insbesondere dann von Vorteil, wenn der Verbraucher in kürzeren Abständen die Farbnuance ändern will und gleichzeitig eine Beeinträchtigung der Haarqualität durch die häufige Einwirkung von Oxidationsmitteln vermeiden möchten; da nicht-oxidative ("direkt-ziehende") Färbemittel unter sehr schonenden Bedingungen appliziert werden können. Da sich bei Tönungen mit jedem Waschvorgang die Färbungen graduell abschwächen können, überdauern je nach verwendetem Produkt und Haartyp solche Färbungen normalerweise maximal 10 bis 15 Haarwäschen. Häufig nutzen Kunden gerade diese Eigenschaften dazu, anschließend neue Nuancen aufzutragen um ein anderes Farbergebnis zu erhalten. Bei beiden Färbemethoden, der oxidativen wie der nicht-oxidativen, ist die Erzielung einer hohen Farbdichte auf den Haaren bei nur geringer Hautanfärbung besonders erwünscht.

Für die Verwendung in Tönungsmitteln haben sich kationische Farbstoffe mit Arylazogruppen oder Heteroarylazogruppen sowie Anthrachinone oder Naphthochinone als besonders geeignet erwiesen, wobei die kationische Gruppe sowohl delokalisiert als auch lokalisiert sein kann. In diesem Zusammenhang seien zum Beispiel die weit verbreiteten "Arianor®-Farbstoffe" genannt, die nicht nur gute färberische Eigenschaften aufweisen, sondern auch eine sehr geringe Hautanfärbung ergeben. Die "Arianor®-Farbstoffe" bestehen überwiegend aus kationischen Azofarbstoffen, lediglich der blaue Farbstoff ("Arianor® Steel-Blue") ist ein Naphthochinonderivat. Besonders problematisch ist bei diesen "Arianor®-Farbstoffen", dass sich die Farbstoffe bisweilen unterschiedlich schnell auswaschen, wodurch sich infolge des stärkeren Auswaschens eines oder mehrerer Farbstoffe der Farbton unkontrolliert verändern kann.

Überraschenderweise wurde nunmehr gefunden, dass bestimmte kationische Naphthyldiazofarbstoffe in hervorragender Weise zur semipermanenten Färbung von Haaren geeignet sind. Weiterhin zeichnen sich diese Farbstoffe durch ihre Kompatibilität zu den Arianor®-Farbstoffen aus und können diese in idealer Weise bezüglich des Farbenspektrums ergänzen. Ebenfalls verfügen die erfindungsgemäßen Farbstoffe über ein sehr gleichmäßiges Aufziehverhalten und eine außerordentliche Deckkraft, so dass sie zur Erzielung von besonderen modischen Effekten alleine oder aber in den Fällen, in denen natürliche Nuancen erzielt werden sollen, in Kombination mit weiteren Farbstoffen der Formel (I) oder mit auf dem Markt verfügbaren direktziehenden Haarfarbstoffen verwendet werden können.

Gegenstand der vorliegenden Erfindung sind daher kationische Naphthyldiazofarbstoffe der allgemeinen Formel (I), 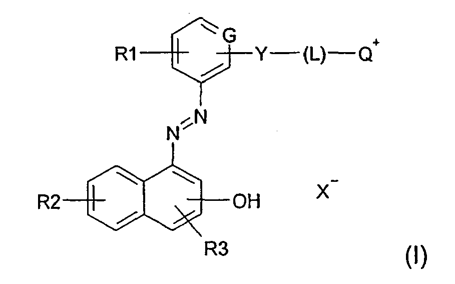 worin
**R1** ein Wasserstoffatom, ein Halogenatom, eine geradkettige oder verzweigte (C1-C4)-Alkylgruppe, eine geradkettige oder verzweigte (C1-C4)-Alkoxygruppe, eine Phenylgruppe oder eine (C2-C4)-Hydroxyalkylgruppe darstellt;
R2 und R3 gleich oder verschieden sein können und unabhängig voneinander ein Wasserstoffatom, eine Hydroxygruppe, eine Aminogruppe, eine Acetylaminogruppe, eine (C1-C6)-Alkoxygruppe, eine (C2-C4)-Hydroxyalkoxygruppe, eine (C3-C6)-Di- oder Polyhydroxyalkoxygruppe, eine -COOR-Gruppe , eine -NRR'-Gruppe oder eine -CONRR'-Gruppe darstellen, wobei R und R' gleich oder verschieden sein können und ein Wasserstoffatom, eine geradkettige oder verzweigte (C1-C6)-Alkylgruppe oder eine Hydroxyethylgruppe darstellen, beziehungsweise R und R' gemeinsam mit dem Stickstoffatom, an das sie gebunden sind, einen Heterozyklus mit mindestens vier Ringgliedern (vorzugsweise einen 4- bis 6-gliedrigen Ring) bilden, der gegebenenfalls weitere Heteroatome wie Sauerstoff, Stickstoff oder Schwefel enthalten kann, wobei R und R' sowie der zuvor beschriebene Heterozyklus mit einer Alkylgruppe, einer Alkoxygruppe, einer Hydroxyalkylgruppe oder einer Aminoalkylgruppe substituiert sein können;
G ein Stickstoffatom oder eine Methingruppe (CH) darstellt;
Y ein Sauerstoffatom oder eine N-(C1-C4)-Alkylgruppe darstellt;
L eine Brückengruppe darstellt und für eine geradkettige oder verzweigte (C1-C14)-Alkylengruppe, die gegebenenfalls durch ein oder mehrere Heteroatome wie Sauerstoff, Stickstoff oder Schwefel unterbrochen sein kann, steht, wobei die Brückengruppe gegebenenfalls mit einer oder mehreren Hydroxygruppen, Monohydroxy-(C2-C6)-alkylgruppen, Polyhydroxy-(C2-C6)-alkylgru,ppen oder (C1-C6)-Alkoxygruppen substituiert ist;
Q⁺ steht für eine gesättigte kationische Gruppe der Formel (II) oder eine ungesättigte kationische Gruppe der Formeln (III) bis (V), 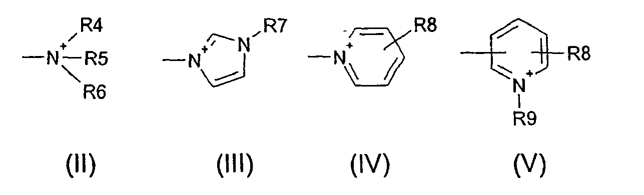 in denen
R4 bis R6 gleich oder verschieden sein können und unabhängig voneinander eine geradkettige oder verzweigte (C1-C6)-Alkylgruppe eine (C2-C4)-Hydroxyalkylgruppe, eine (C3-C6)-Dihydroxyalkylgruppe, eine (C3-C6)-Polyhydroxyalkylgruppe oder eine (C1-C6)-Alkoxy-(C1-C4)-alkylgruppe darstellen, wobei zwei der Gruppen R4 bis R6 zusammen mit dem Stickstoffatom, an das sie gebunden sind, einen fünf- oder sechsgliedrigen Heterozyklus bilden, der gegebenenfalls durch ein oder mehrere Heteroatome wie ein Sauerstoffatom, ein Schwefelatom oder ein Stickstoffatom unterbrochen sein kann und gegebenenfalls weitere Substituenten, wie beispielsweise ein Halogenatom, eine Hydroxygruppe, eine Aminogruppe, eine geradkettige oder verzweigte (C1-C6)-Alkylgruppe, eine (C1-C6)-Alkoxygruppe, eine (C1-C6)-Alkoxy-(C1-C4)-alkylgruppe oder eine Hydroxyethylgruppe aufweisen kann;
R7 eine geradkettige oder verzweigte (C1-C8)-Alkylgruppe, eine Allylgruppe, eine Vinylgruppe, eine Hydroxyethylgruppe oder eine Benzylgruppe darstellt;
**R8** ein Wasserstoffatom, eine geradkettige oder verzweigte (C1-C9)-Alkylgruppe, eine Aminogruppe, eine Di-(C1-C6)-alkylaminogruppe oder eine Pyrrolidinogruppe darstellt;
**R9** eine geradkettige oder verzweigte (C1-C8)-Alkylgruppe, eine Allylgruppe, eine Vinylgruppe, eine Hydroxyethylgruppe, eine Dihydroxypropylgruppe oder eine Benzylgruppe darstellt; und
X⁻ für ein Anion, vorzugsweise ein Chloridanion, Bromidanion, Jodidanion, Alkylsulfatanion, Arylsulfonatanion, Hydrogensulfatanion, Sulfatanion, Phosphatanion, Acetatanion oder Tartratanion, steht.

Bevorzugt sind Farbstoffe der Formel (I), in denen
**R1** ein Wasserstoffatom, ein Chloratom oder eine Methylgruppe darstellt,
R2 und R3 gleich oder verschieden sind und unabhängig voneinander Wasserstoff, eine Hydroxygruppe, eine Methoxygruppe, eine -NRR'-Gruppe oder eine -CONRR'-Gruppe darstellen, wobei **R** und **R'** gleich oder verschieden sein können und ein Wasserstoffatom, eine Methylgruppe oder eine Hydroxyethylgruppe darstellen, oder aber R und R' gemeinsam mit dem Stickstoffatom, an das sie gebunden sind, einen Heterozyklus mit fünf oder sechs Ringgliedern bilden -beispielsweise eine Pyrrolidinogruppe, eine 3-Hydroxy-pyrrolidinogruppe, eine 2-Methoxymethyl-pyrrolidinogruppe, eine 2,5-Bis-(methoxymethyl)-pyrrolidinogruppe, eine Piperidinogruppe, eine 3-Hydroxy-piperidinogruppe, eine 4-Hydroxy-piperidinogruppe, eine 4-Methyl-piperidinogruppe, eine 2,3- oder 2,6-Dimethyl-piperidinogruppe, eine 2-Ethyl-piperidinogruppe, eine 4-Benzyl-piperidinogruppe, eine Morpholinogruppe, eine N-Methyl-piperazinogruppe oder eine 1-(2-Hydroxyethyl)-piperazinogruppe-;
G für ein Stickstoffatom oder eine Methingruppe (CH) steht;
Y gleich Sauerstoff oder einer N-Methylgruppe ist;
L für eine geradkettige (C2-C4)-Brückengruppe steht;
**Q⁺** eine gesättigte kationische Gruppe der Formel (II) oder eine ungesättigte kationische Gruppe der Formel (III) bis (V) darstellt, wobei die Restgruppen R4 bis R6 gleich oder verschieden sein können und unabhängig voneinander eine geradkettige (C1-C3)-Alkylgruppe, eine Hydroxyethylgruppe oder eine Methoxyethylgruppe darstellen, oder aber zwei der Gruppen R4 bis R6 gemeinsam mit dem Stickstoffatom an das sie gebunden sind einen fünfgliedrigen oder sechsgliedrigen Heterozyklus bilden, beispielsweise eine Pyrrolidinogruppe, eine 3-Hydroxy-pyrrolidinogruppe, eine 2-Methoxymethyl-pyrrolidinogruppe, eine 2,5-Bis-(methoxymethyl)-pyrrolidinogruppe, eine Piperidinogruppe, eine 3-Hydroxy-piperidinogruppe, eine 4-Hydroxy-piperidinogruppe, eine 2-Methyl-piperidinogruppe, eine 3-Methyl-piperidinogruppe, eine 4-Methyl-piperidinogruppe, eine 2,6-Dimethyl-piperidinogruppe, eine 2-Ethyl-piperidinogruppe, eine 4-Benzyl-piperidinogruppe, eine Morpholinogruppe, eine N-Methyl-piperazinogruppe oder eine 1-(2-Hydroxyethyl)-piperazinogruppe;
R7 eine Methylgruppe oder eine Hydroxyethylgruppe darstellt;
R8 ein Wasserstoffatom, eine Methylgruppe, eine Dimethylaminogruppe oder eine Pyrrolidinogruppe darstellt;
R9 gleich einer Methylgruppe, einer Ethylgruppe oder einer Hydroxyethylgruppe ist und
X⁻ gleich einem Chloridanion, einem Bromidanion oder einem Methylsulfatanion ist.

Besonders bevorzugte Farbstoffe der Formel (I) sind 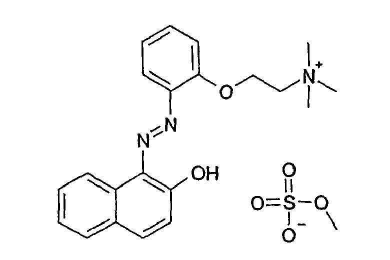 2-{2-[(2-Hydroxy-1-naphthyl)diazenyl]phenoxy}-N,N,N-trimethyl-ethanaminium-methylsulfat **(1)** 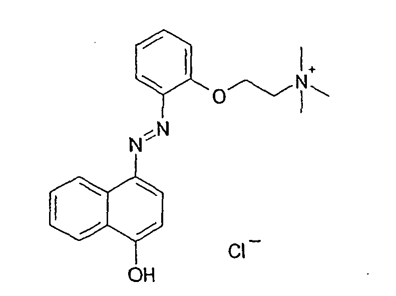 2-{2-(4-Hydroxy-1-naphthyl)diazenyl]phenoxy}-N,N,N-trimethyl-ethanaminium-chlorid (2) 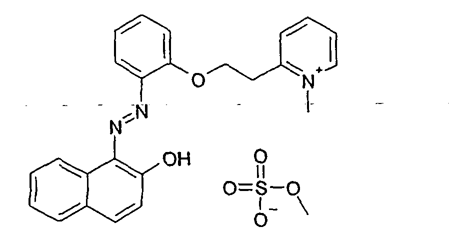 2-(2-{2-[(2-Hydroxy-1-naphthyl)diazenyl]phenoxy}ethyl)-1-methylpyridinium-methylsulfat (3) 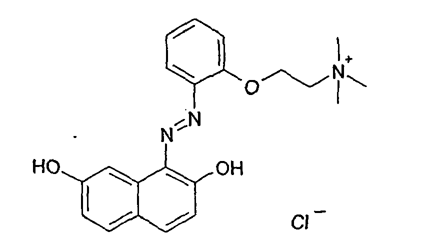 2-{2-[(2,7-Dihydroxy-1-naphthyl)diazenyl]phenoxy}-N,N,N-trimethyl-ethanaminium-chlorid (4) 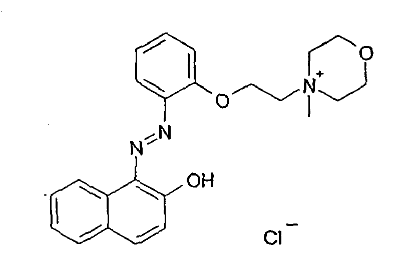 4-(2-{2-[(2-Hydroxy-1-naphthyl)diazenyl]phenoxy}ethyl)-4-methylmorpholin-4-ium-chlorid (5) 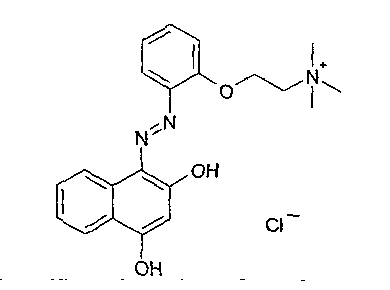 2-({2-[(2,4-Dihydroxy-1-naphthalenyl)diazenyl]-phenyl}oxy)-N,N,N-trimethylethanaminium-chlorid (6) 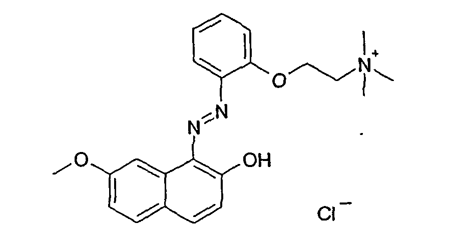 2-[(2-{[2-Hydroxy-7-(methyloxy)-1-naphthatenyl]diazenyl}phenyl)oxy]-N,N,N-trimethylethanaminium-chlorid (7) 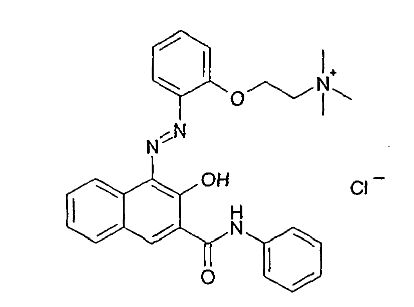 2-{[2-({2-Hydroxy-3-[(phenylamino)carbonyl]-1-naphthalenyl}diazenyl)-phenyl]oxy}-N,N,N-trimethylethanaminium-chlorid (8) 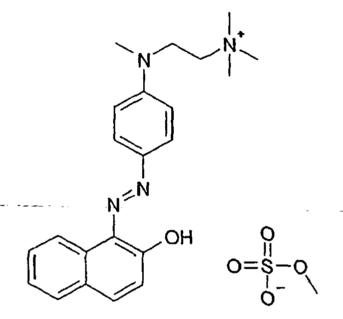 2-[{4-[(2-Hydroxy-1-naphthalenyl)diazenyl]phenyl}-(methyl)amino]-N,N,N-trimethylethanaminium-methylsulfat (9) 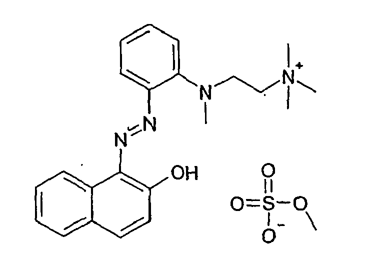 2-[{2-[(2-Hydroxy-1-naphthatenyl)diazenyl]-phenyl}(methyl)amino]-N,N,N-trimethylethanaminium-methylsulfat (10) 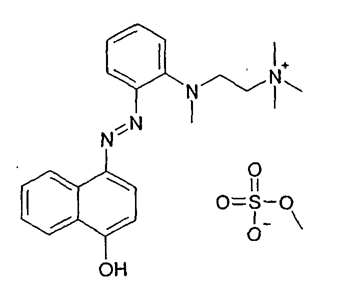 2-[{2-[(4-Hydroxy-1-naphthalenyl)diazenyl]-phenyl}(methyl)amino]-N,N,N-trimethylethanaminium-methylsulfat **(11)** 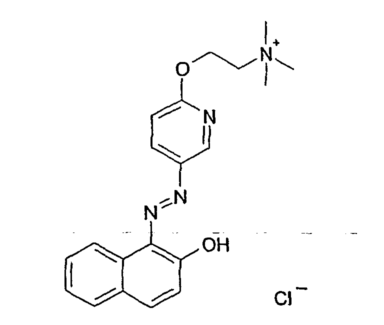 2-({5-[(2-Hydroxy-1-naphthyl)diazenyl]-2-pyridinyl}oxy)-N,N,N-trimethylethanaminium-chlorid (12) 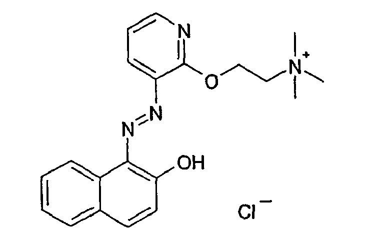 2-({3-[(2-Hydroxy-1-naphthyl)diazenyl]-2-pyridinyl}oxy)-N,N,N-trimethylethanaminium-chlorid (13) 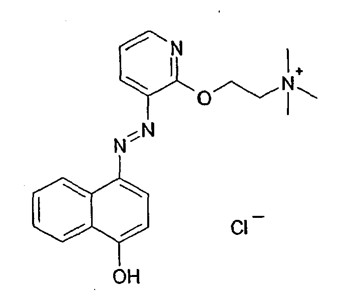 2-({3-[(4-Hydroxy-1-naphthyl)diazenyl]-2-pyridinyl}oxy)-N,N,N-trimethylethanaminium-chlorid (14) 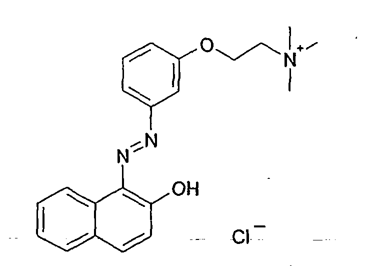 2-{3-[(2-Hydroxy-1-naphthyl)diazenyl]phenoxy}-N,N,N-trimethylethanaminium-chlorid (15) 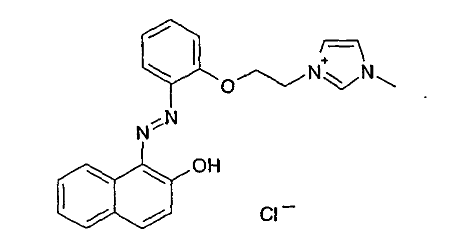 3-(2-{2-[(2-Hydroxy-1-naphthyl)diazenyl]phenoxy}ethyl)-1-methyl-1*H-*imidazol-3-ium chlorid **(16)**

Die erfindungsgemäßen Farbstoffe der Formel (1) werden vorzugsweise nach Schema 1 hergestellt, wobei als Ausgangsverbindungen insbesondere kationische Nitroaromaten der allgemeinen Formel (VI), wie sie beispielsweise in der Patentschrift DE-A 2017497 beschrieben werden, verwendet werden. Nach der Reduktion der Nitroverbindungen, beispielsweise mittels katalytischer Hydrierung, erhält man die Amine der Formel (VII), die nach Standardmethoden, vorzugsweise in Wasser, diazotiert und auf die entsprechenden 1- oder 2-Naphtholderivate gekuppelt werden.

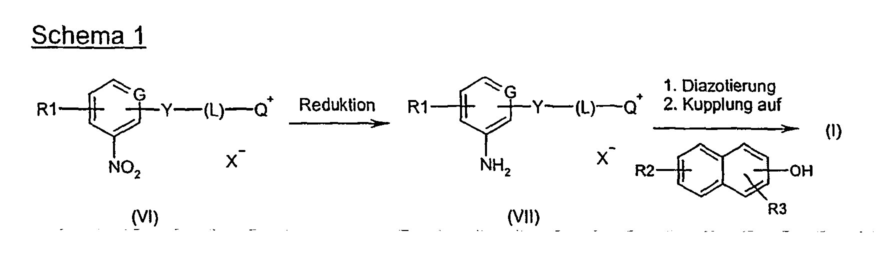

Die erfindungsgemäßen Farbstoffe der Formel (I) eignen sich hervorragend für den Einsatz in Färbemitteln für Keratinfasern, insbesondere Haare.

Ein weiterer Gegenstand der vorliegenden Anmeldung sind daher Färbemittel für Keratinfasern, enthaltend mindestens einen kationischen Naphthyldiazofarbstoff der allgemeinen Formel (I).

Diese Färbemittel enthalten die kationischen Naphthyldiazofarbstoffe der allgemeinen Formel (I) in einer Gesamtmenge von 0,01 bis 10 Gewichtsprozent, insbesondere 0,1 bis 6 Gewichtsprozent.

Neben den Farbstoffen der Formel (I) kann das Färbemittel weitere Farbstoffe, insbesondere 3-((4,5-Dihydro-3-methyl-5-oxo-1-phenyl-1H-pyrazol-4-yl)azo)-N,N,N-trimethyl-benzen-aminiumchlorid, 3-[(3-Methyl-5-hydroxy-1-phenyl-1 H-pyrazol-4-yl)azo]-trimethylammonio-benzol-chlorid (C.l. 12719; Basic Yellow 57), 8-[(4-Aminophenyl)azo]-7-hydroxy-N,N,N-trimethyl-2-naphthalinaminium-chlorid (C.l. 12250; Basic Brown 16), 8-[(4-Amino-3-nitrophenyl)azo]-7-hydroxy-N,N,N-trimethyl-2-naphthalin-aminium-chlorid (C.l. 12251; Basic Brown 17), 8-[(4-Amino-2-nitrophenyl)-azo]-7-hydroxy-N,N,N-trimethyl-2-naphthalinaminium-chlorid (C.l. 12251:1; Basic Red 118), 7-Hydroxy-N,N,N-trimethyl-8-{[2-(methyloxy)phenyl]-azo}-2-naphthalinaminium-chlorid (C.l. 12245; Basic Red 76), 3-((4-Amino-6-bromo-5,8-dihydro-1-hydroxy-8-imino-5-oxo-2-naphthalenyl)amino)-N,N,N-trimethyl-benzolamoniumchlorid (C.l. 56059; Basic Blue 99) und N,N-Dimethyl-3-{[4-(methylamino)-9,10-dioxo-9,10-dihydro-1-anthracenyl]-amino}-N-propyl-1-propanaminium-bromid, enthalten.

Zur Erweiterung der Farbpalette kann das erfindungsgemäße Färbemittel zusätzlich weitere natürliche oder synthetische nicht-oxidative Farbstoffe enthalten. Als natürliche Farbstoffe können Pflanzenfarbstoffe wie zum Beispiel Henna oder Indigo genannt werden, während als synthetische nicht-oxidative Farbstoffe beispielsweise andere kationische Farbstoffe welche nicht unter die zuvor genannte Formel (I) fallen, Nitrofarbstoffe, Azofarbstoffe, Triphenylmethanfarbstoffe oder Chinonfarbstoffe genannt werden. Als weitere natürliche oder synthetische nicht-oxidative Farbstoffe können insbesondere die nachfolgend genannten direktziehenden Farbstoffe eingesetzt werden:
9-(Dimethylamino)-benzo[a]phenoxazin-7-ium-chlorid (C.I. 51175; Basic Blue 6), Di[4-(diethylamino)phenyl][4-(ethylamino)naphthyl]carbenium-chlorid (C.I. 42595; Basic Blue 7), 3,7-Di(dimethylamino)phenothiazin-5-ium-chlorid (C.I. 52015; Basic Blue 9), Di[4-(dimethylamino)phenyl]-[4-(phenyl-amino)naphthyl]carbenium-chlorid (C.I. 44045; Basic Blue 26), 2-[(4-(Ethyl(2-hydroxyethyl)amino)-phenyl)azo]-6-methoxy-3-methylbenzothiazolium-methylsulfat (C.I. 11154; Basic Blue 41), 2,8-Dimethyl-7-(dimethylamino)-3-imino-3H-phenoxazin-monohydrochlorid (Basic Blue 151), 7-(Dimethylamino)-3-imino-2-methoxy-3H-phenoxazin-monohydrochlorid (Basic Blue 124), Bis[4-(dimethylamino)-phenyl]-[4-(methylamino)phenyl]carbenium-chlorid (C.I. 42535; Basic Violet 1), 4-[(4-Amino-3-methylphenyl)(4-imino-3-methyl-2,5-cyclohexadien-1-ylidene)methyl]-2-methylbenzenamin-hydrochlorid (C.I. 42520, Basic Violet 2), Tris[4-(dimethylamino)phenyl]-carbenium-chlorid (C.I. 42555; Basic Violet 3), 2-[3,6-(Diethylamino)-dibenzopyranium-9-yl]-benzoesäure-chlorid (C.I.45170; Basic Violet 10), Di(4-aminophenyl)-(4-amino-3-methylphenyl)carbenium-chlorid (C.I. 42510; Basic Violet 14), 1,3-Bis[(2,4-diamino-5-methylphenyl)azo]-3-methylbenzol (C.I. 21010; Basic Brown 4), 3,7-Diamino-2,8-dimethyl-5-phenylphenazinium-chlorid (C.I. 50240; Basic Red 2), 1,4-Dimethyl-5-[(4-(dimethylamino)phenyl)azo]-1,2,4-triazolium-chlorid (C.I. 11055; Basic Red 22), 3(oder5)-[[4-[Benzylmethylamino]phenyl]azo]-1,2-(oder1,4)-dimethyl-1,2,4-triazolium-bromid (C.I. Basic Red 46), 2-{[4-(Dimethylamino)phenyl]azo}-1,3-dimethyl-1 H-imidazol-3-ium-chlorid (Basic Red 51), 5-{[4-(Dimethylamino)phenyl]azo}-1 ,2-dimethyl-1H-pyrazol-2-ium-chlorid, 1,3-Dimethyl-2-{[4-(methylamino)-phenyl]azo}-1 H-imidazol-3-ium-chlorid (Basic Red 109), 2-[(4-Aminophenyl)azo]-1,3-dimethyl-1H-imidazol-3-ium-chlorid, 4-{[4-(Dimethylamino)phenyl]azo}-1-methylpyridinium-chlorid, N,N-Dimethyl-4-[(E)-(1-oxido-4-pyridinyl)diazenyl]anilin oder 4-((4-(Dimethylamino)phenyl)azo)-pyridin-N-oxid, 2-[2-((2,4-Dimethoxyphenyl)amino)ethenyl]-1,3,3-trimethyl-3H-indol-1-ium-chlorid (C.l. 48055; Basic Yellow 11), 1-Methyl-4-{[methyl-(phenyl)hydrazono]methyl}pyridinium-chlorid (Basic Yellow 87), 1-Methyl-4-{(E)-[methyl(4-methoxy-phenyl)hydrazono]methyl}pyridinium-chlorid, 1-Methyl-4-({methyl[4-methoxy-phenyl]hydrazono}methyl)pyridinium-methylsulfat (Basic Yellow 91) oder Bis[4-(diethylamino)phenyl]-phenyl-carbenium-hydrogensulfat (1:1) (C.I. 42040; Basic Green 1).

Obwohl die in dem erfindungsgemäßen Mittel enthaltenen Farbstoffe der Formel (1) kationischen Charakter besitzen, können diese je nach verwendeter Farbträgermasse in speziellen Fällen auch in Kombination mit anionischen ("sauren") Farbstoffen verwendet werden.

Der Gesamtgehalt an weiteren (insbesondere natürlichen und/oder synthetischen nicht-oxidativen) Farbstoffen in dem erfindungsgemäßen Färbemittel beträgt etwa 0,01 bis 15 Gewichtsprozent, insbesondere etwa 0,1 bis 12 Gewichtsprozent.

Zur Erhöhung der Farbintensität können die in kosmetischen Systemen üblichen Carrier zugesetzt werden. Geeignete Verbindungen werden zum Beispiel in der DE-A 196 18 595 beschrieben, auf die hiermit ausdrücklich Bezug genommen wird. Besonders geeignete Carrier sind zum Beispiel Benzylalkohol, Vanillin und Isovanillin.

Das erfindungsgemäße Mittel zur Färbung von Keratinfasern kann beispielsweise in Form einer Lösung, insbesondere als wäßrigalkoholische Lösung, einer Creme, eines Geles oder einer Emulsion vorliegen. Als Lösungsmittel können neben Wasser beispielsweise niedere aliphatische einwertige oder mehrwertige Alkohole, deren Ester und Ether, oder aber Gemische dieser Lösungsmittel untereinander oder mit Wasser genannt werden. Der maximale Siedepunkt der vorgenannten geeigneten Lösungsmittel beträgt etwa 400 °C, wobei ein Siedepunkt von 20 °C bis 250 °C bevorzugt ist.

Ebenfalls ist es möglich, das erfindungsgemäße Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder als Aerosolschaum aus einem Druckbehälter zu entnehmen.

Der pH-Wert des erfindungsgemäßen Färbemittels beträgt etwa 2 bis 11, wobei ein pH-Wert von etwa 5 bis 9 besonders bevorzugt ist. Die Einstellung eines alkalischen pH-Wertes erfolgt vorzugsweise mit Ammoniak, es ist jedoch auch möglich, anstelle von Ammoniak organische Amine, wie zum Beispiel Monoethanolamin oder Triethanolamin zu verwenden. Für die Einstellung eines sauren pH-Wertes kann hingegen eine organische oder anorganische Säure, wie zum Beispiel Salzsäure, Schwefelsäure, Phosphorsäure, Ascorbinsäure, Glycolsäure oder Milchsäure, verwendet werden.

Selbstverständlich kann das vorstehend beschriebene Färbemittel gegebenenfalls weitere für Färbemittel für Keratinfasern übliche Zusätze, wie zum Beispiel Pflegestoffe, Netzmittel, Verdicker, Weichmacher, Konservierungsstoffe und Parfümöle sowie auch weitere, nachstehend aufgeführte Zusätze enthalten.

Weiterhin können in dem erfindungsgemäßen Färbemittel Netzmittel oder Emulgatoren aus den Klassen der anionischen, amphoteren, nichtionogenen oder zwitterionischen oberflächenaktiven Substanzen wie Fettalkoholsulfate, Alkansulfonate, Alkylbenzolsulfonate, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein.

Das vorstehend beschriebene Färbemittel kann weiterhin natürliche oder synthetische Polymere beziehungsweise modifizierte Polymere natürlichen Ursprungs enthalten, wodurch gleichzeitig mit der Färbung eine Festigung der Keratinfaser erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent. Die vorgenannten Polymere können in dem erfindungsgemäßen Mittel in der für solche Mittel üblichen Menge, insbesondere in einer Menge von etwa 1 bis 5 Gewichtsprozent, verwendet werden.

Das erfindungsgemäße Mittel zur Färbung von Keratinfasern eignet sich insbesondere zu Färbung von Haaren. Das erfindungsgemäße Färbemittel wird hierzu in üblicher Weise in einer für die Färbung der Haare ausreichenden Menge, im allgemeinen etwa 50 bis 150 Gramm, auf das Haar aufgetragen. Nach einer für die Haarfärbung ausreichenden Einwirkungszeit, die üblicherweise etwa 10 bis 45 Minuten bei 20 bis 50 °C, vorzugsweise etwa 15 bis 30 Minuten bei etwa 40 °C, beträgt, wird das Haar mit Wasser gespült, gegebenenfalls mit einem Shampoo gewaschen und/oder mit der wäßrigen Lösung einer schwachen organischen Säure, wie zum Beispiel Zitronensäure oder Weinsäure, nachgespült und getrocknet.

Die Anwendung des Färbemittels mit zusätzlicher Festigung erfolgt in bekannter und üblicher Weise durch Befeuchten des Haares mit dem Festiger, Einlegen des Haares zur Frisur und anschließende Trocknung.

Mit dem erfindungsgemäßen Färbemittel lassen sich Nuancen sowohl im Naturtonbereichals auch im modischen Bereich (zum Beispiel hochmodische, leuchtende Nuancen) erzielen. Darüber hinaus ist es möglich, neben den erwähnten Tönen eine Reihe von brillanten Farbreflexen über den gesamten sichtbaren Farbbereich zu erzielen. Bedingt durch die sehr hohe Farbstärke der Farbstoffe und das gute Aufziehverhalten kann in hervorragender Weise der ursprüngliche Farbton der Faser überdeckt werden. Damit kann auch dem eingangs erwähnten Wunsch nach der Integration der Haarfarbe in die Mode sowie als Ausdruck der Persönlichkeit voll entsprochen werden. Die vorzüglichen Eigenschaften des neuen Färbemittels zeigen sich insbesondere auf durch Licht und Wetter geschädigtem oder auf dauergewellten Haaren. Hierbei zeichnen sich die erhaltenen Färbungen insbesondere auch durch ihre sehr gute Haltbarkeit und Auswaschbeständigkeit aus.

Die nachstehenden Beispiele sollen den Erfindungsgegenstand näher erläutern, ohne ihn auf die angeführten Beispiele zu beschränken.

### Beispiele

### Beispiel 1: Herstellung von 2-{2-[(2-hydroxy-1-naphthyl)diazenyl]-phenoxy}-N,N,N-trimethylethanaminium-methylsulfat

### Stufe 1.1: Herstellung von 2-(2-Aminophenoxy)-N,N,N-trimethyl-ethanaminium-methylsulfat

34 g (100 mmol) N,N,N-Trimethyl-2-(2-nitrophenoxy)ethanaminium-methylsulfat gemäß DE-A 20 17 497 werden in 300 ml Ethanol an 3,4 g Pd/C (10%ig) bei 9 bar Wasserstoffdruck hydriert. Nach 2 Stunden wird vom Katalysator abfiltriert und zur Trockne eingedampft. Man erhält ein farbloses Öl, das allmählich erstarrt.
Ausbeute: 31 g (100 % der Theorie)
¹H-NMR (DMSO-*d₆*): δ = 6,85 ppm (d, 1H); 6,70 ppm (m, 2H); 6,52 ppm (m, 1 H); 4,82 ppm (s breit, 2H); 4,39 ppm (s breit, 2H); 3,79 ppm (s breit, 2H); 3,38 ppm (s, 3H); 3,19 ppm (s, 9H).

### Stufe 1.2: Herstellung von 2-{2-[(2-Hydroxy-1-naphthyl)diazenyl]-phenoxy}-N,N,N-trimethylethanaminium-methylsulfat

15,6 g (50 mmol) der Stufe 9.1 werden in 160 ml Wasser und 18,3 g einer 32%igen Salzsäurelösung gelöst und unter Kühlung im Eisbad mit 4,2 g (60 mmol) Natriumnitrit in 25 ml Wasser diazotiert, wobei die Temperatur 5 °C nicht überschreiten sollte. Es wird noch 1 Stunde lang weitergerührt und anschließend der Nitritüberschuß durch Zugabe von-Sulfaminsäure zersetzt. Man gießt die Diazoniumsalzlösung unter Rühren in eine Lösung von 7,4 g (51 mmol) 2-Naphthol in 50 ml Isopropanol. Durch Zugabe einer gesättigten Natriumcarbonatlösung stellt man den pH-Wert auf 9 bis 10 ein, wobei Kupplung eintritt und ein dicker Farbstoffbrei entsteht. Es wird noch 30 Minuten lang weitergerührt und abgesaugt. Das Rohprodukt wird anschließend in 300 ml 60 °C warmem Wasser aufgenommen, mit 2normaler Salzsäure neutralisiert, und nach Abkühlung erneut abgesaugt. Nach dem Trocknen erhält man 13 g (55 % der Theorie) rotes Produkt.
¹H-NMR (DMSO-d₆): δ = 8,51 (d, 1 H); 8,08 (d, 1 H); 7,91 (d, 1 H); 7,73 (d, 1 H); 7,58 (t, 1H); 7,43 (t, 1 H); 7,37 (m, 2H); 7,22 (m, 1 H); 6,80 (d, 1 H); 4,72 (t, 2H); 3,96 (t, 2H); 3,32 ppm (s, 12H).

### Beispiel 2: Herstellung von 2-{2-[(4-Hydroxy-1-naphthyl)diazenyl]-phenoxy}-N,N,N-trimethylethanaminium-chlorid

Man verfährt wie in Beispiel 1 beschrieben, wobei anstelle von 2-Naphthol die gleiche Menge 1-Naphthol verwendet wird.
Ausbeute: 11,8 g (50 % der Theorie)
¹H-NMR (DMSO-*d₆*): δ = 8,77 ppm (d, 1 H); 8,22 ppm (dd, 1 H); 7,86.ppm (d, 1 H); 7,72 ppm (dd, 1 H); 7,45 ppm (m, 1 H); 7,26 ppm (m, 2H); 7,20 ppm (d, 1 H); 7,08 ppm (m, 1 H); 6,41 ppm (d, 1 H); 4,60 ppm (t, 2H); 3,88 ppm (t, 2H); 3,28 ppm (s, 9H).

### Beispiel 3: Herstellung von 2-(2-{2-[(2-Hydroxy-1-naphthyl)diazenyl]-phenoxy}ethyl)-1-methylpyridinium-methylsulfat

### Stufe 3.1: Herstellung von 2-[2-(2-Nitrophenoxy)ethyl]pyridin

7,05 g (50 mmol) 2-Fluor-nitrobenzol, 6,77 g (50 mmol) 2-(2-Hydroxyethyl)-pyridin und 16,62 g (50 mmol) Cäsiumcarbonat werden in 30 ml Dimethylformamid 7 Tage lang bei Raumtemperatur gerührt. Anschließend wird filtriert und das Filtrat fraktioniert destilliert. Das Lösungsmittel und nicht umgesetztes 2-Fluor-nitrobenzol destillieren unterhalb von 100 °C bei 8 bis 4 mbar. Das Produkt destilliert in einer Kugelrohrdestille bei 170 bei 180 °C Ofentemperatur und einem Vakuum von 3 mbar.
Ausbeute: 8,7 g (71 % der Theorie).
¹H-NMR (DMSO-d₆): δ = 8,50 ppm (d, 1 H); 7,82 ppm (dd, 1 H); 7,69 ppm (dt, 1 H); 7,61 ppm (dt, 1H); 7,36 ppm (m, 2H); 7,22 ppm (t, 1 H); 7,07 ppm (t, 1 H); 4,53 ppm (t, 2H); 3,20 ppm (t, 2H).

### Stufe 3.2: Herstellung von 1-Methyl-2-[2-(2-nitrophenoxy)ethyl]-pyridinium-methylsulfat

4,88 g (20 mmol) der Stufe 3.1 werden in 60 ml Essigsäureethylester gelöst und unter Rühren mit 2,78 g (20 mmol) Dimethylsulfat versetzt. Allmählich bildet sich ein farbloser Niederschlag. Man rührt noch 4 Stunden langen bei Raumtemperatur weiter, filtriert ab, wäscht mit wenig Essigsäureethylester nach und trocknet anschließend im Vakuum bei 40°C.
Ausbeute: 6 g (81 % der Theorie) farbloses Produkt
¹H-NMR (DMSO-*d₆*): δ = 9,06 ppm (d, 1 H); 8,55 ppm (dt, 1 H); 8,12 ppm (d, 1 H); 8,02 ppm (dt, 1 H); 7,71 ppm (dd, 1 H); 7,69 ppm (dt, 1 H); 7,42 ppm (d, 1 H); 7,15 ppm (dt, 1 H); 4,65 ppm (t, 2H); 4,37 ppm (s, 3H); 3,65 ppm (t, 2H); 3,37 ppm (s, 3H).

### Stufe 3.3: Herstellung von 2-[2-(2-Aminophenoxy)ethyl]-1-methylpyridinium-methylsulfat

3,7 g (10 mmol) der Stufe 3.2 werden in 25 ml Wasser an 0,3 g Pd/C (10%ig) bei 9 bar Wasserstoffdruck hydriert. Nach 2 Stunden wird vom Katalysator abfiltriert und die erhaltene Lösung direkt weiter umgesetzt.

### Stufe 3.4: Herstellung von 2-(2-{2-[(2-Hydroxy-1-naphthyl)diazenyl]-phenoxy}-ethyl)-1-methylpyridinium-methylsulfat

Die Lösung aus Stufe 3.3 (10 mmol) wird mit 2,9 g einer 32%igen Salzsäurelösung versetzt. Anschließend werden im Eisbad 0,7 g (10 mmol) Natriumnitrit, gelöst in 5 ml Wasser, innerhalb von 10 Minuten zugetropft, wobei die Temperatur 5 °C nicht übersteigen sollte. Man rührt noch 1 Stunde lang im Eisbad weiter und zersetzt dann nicht umgesetztes Nitrit durch Zugabe von Sulfaminsäure. Diese Lösung gibt man zu einer Lösung aus 1,45 g 2-Naphthol (10 mmol) in 15 ml Wasser, 15 ml Isopropanol und 3,4 g einer 30%igen Natronlauge. Die Azokupplung findet spontan statt, wobei ein dicker roter Brei entsteht. Man läßt noch 1 Stunde lang weiterrühren und saugt ab. Der erhaltene Farbstoff wird bei 40 °C im Vakuum getrocknet.
Ausbeute: 3,65 g (95 % der Theorie) dunkelrotes Produkt
¹H-NMR (DMSO-*d₆*): δ = 8,51 ppm (m, 1H); 8,49 ppm (d, 1H); 8,04 ppm (d, 1H); 7,92 ppm (d, 1H); 7,70 ppm (m, 2H); 7,56 ppm (m, 2H); 7,47 ppm (t, 1H); 7,29 ppm (m, 2H); 7,23 ppm (m, 1H); 7,12 ppm (m, 1H); 6,84 ppm (d, 1H); 4,56 ppm (t, 2H); 3,33 ppm (t, Signalüberlagerung mit Wasser, 2H).

### Beispiel 4: Herstellung von 2-{2-[(2,7-Dihydroxy-1-naphthyl)-diazenyl]-phenoxy}-N,N,N-trimethylethanaminium-chlorid

Man verfährt wie in Synthesebeispiel 1 beschrieben, wobei anstehe von 2-Naphthol 8,0 g (50 mmol) 2,7-Dihydroxynaphthalin verwendet werden.
Ausbeute: 16,10 g (80 % der Theorie) dunkelrotes Produkt
¹H-NMR (DMSO-*d₆**)*: δ = 10,12 ppm (s, 1 H); 8,00 ppm (d, 1 H); 7,81 ppm (d, 1 H); 7,80 ppm (d, 1 H); 7,56 ppm (d, 1 H); 7,32 ppm (m, 2H); 7,22 ppm (t, 1 H); 6,91 ppm (dd, 1 H); 6,53 ppm (d, 1 H); 4,70 ppm (t, 2H); 3,95 ppm (t, 2H); 3,37 ppm (s, 9H).

### Beispiel 5: Herstellung von 4-(2-{2-[(2-Hydroxy-1-naphthyl)diazenyl]-phenoxy}ethyl)-4-methylmorpholin-4-ium-chlorid

### Stufe 5.1: Herstellung von 4-[2-(2-Nitrophenoxy)ethyl]morpholin

In Analogie zur WO 00/59883, Beispiel 1, werden 14,11 g (100 mmol) 2-Fluornitrobenzol, 14,45 g (100 mmol) Morpholinoethanol, 35,8 g (100 mmol) Cäsiumcarbonat und 100 ml Dimethylformamid 7 Tage lang bei Raumtemperatur gerührt. Man filtriert, zieht das Dimethylformamid am Rotationsverdampfer ab und destilliert anschließend den Rückstand im Kugelrohrofen bei 4 mbar und 160 bis 170 °C Ofentemperatur.
Ausbeute: 17,8 g (70,6 % der Theorie) gelbliches Öl
¹H-NMR (DMSO-*d₆*): δ = 7,85 ppm (d, 1H); 7,64 ppm (t, 1H); 7,38 ppm (d, 1H); 7,11 ppm (t, 1H); 4,27 ppm (t, 2H); 3,55 ppm (m, 4H); 2,70 ppm (t, 2H).

### Stufe 5.2: Herstellung von 4-Methyl-4-[2-(2-nitrophenoxy)ethyl]-morpholin-4-ium-methylsulfat

2,52 g (10 mmol) Stufe 5.1 werden in 30 ml Essigsäureethylester gelöst, mit 1,4 g (11 mmol) Dimethylsulfat versetzt und bei Raumtemperatur gerührt, wobei ein Niederschlag entsteht. Man filtriert nach 4 Stunden ab und wäscht mit wenig Essigester nach. Nach dem Tocknen bei 40 °C im. Vakuum erhält man 2,3 g (61 % der Theorie) eines beigefarbenen, leicht klebrigen Produktes.
¹H-NMR (DMSO-*d*₆): δ = 7,94 ppm (dd, 1 H); 7,73 ppm (dt, 1 H); 7,42 ppm (d, 1 H); 7,20 ppm (dt, 1 H); 4,69 ppm (t, 2H); 4,02 ppm (t, 2H); 3,96 ppm (m, 4H); 3,55 ppm (m, 4H); 3,38 ppm (s, 3H); 3,30 ppm (s, 3H).

### Stufe 5.3: Herstellung von 4-[2-(2-Aminophenoxy)ethyl]-4-methylmorpholin-4-ium-methylsulfat

2,08 g (5,5 mmol) der Verbindung aus Stufe 5.2 werden in 80 ml Ethanol gelöst und an 0,25 g Pd/C (10%ig) bei 9 bar Wasserstoffdruck hydriert. Nach 2 Stunden wird vom Katalysator abfiltriert und das Filtrat zur Trockne eingedampft. Man erhält 1,9 g (99 % der Theorie) farbloses Öl, das ohne weitere Reinigung umgesetzt wird.

### Stufe 5.4: Herstellung von 4-(2-{2-[(2-Hydroxy-1-naphthyl)diazenyl]-phenoxy}ethyl)-4-methylmorpholin-4-ium-chlorid

1,5 g (4,3 mmol) der Verbindung aus Stufe 5.3 werden in 30 ml Wasser und 1,3 g einer 32%igen Salzsäurelösung gelöst. Bei 0 bis 5 °C werden 0,31 g (4,5 mmol) Natriumnitrit in 3 ml Wasser zugetropft, so dass die Temperatur 5 °C nicht überschreitet. Nach 1 Stunde Nachrühren im Eisbad und Zersetzen des Nitritüberschusses mit Sulfaminsäure gießt man unter Rühren in eine Lösung von 0,65 g (4,3 mmol) 2-Naphthol in 8 ml Isopropanol. Man gibt zu dieser Mischung eine gesättigte Natriumcarbonatösung bis der pH-Wert auf 9 eingestellt ist. Man läßt noch 30 Minuten weiterrühren, neutralisiert anschließend mit verdünnter Salzsäure und saugt ab. Das so erhaltene Rohprodukt wird 15 Minuten bei Raumtemperatur in 20 ml 2normaler Salzsäure suspendiert, abgesaugt und getrocknet.
Ausbeute: 1,75 g (95 % der Theorie) roter Farbstoff
¹H-NMR (DMSO-*d₆*): δ = 8,50 ppm (d, 1 H); 8,11 ppm (d, 1 H); 7,96 ppm (d, 1 H); 7,76 ppm (d, 1 H); 7,61 ppm (t, 1 H); 7,49 ppm (t, 1 H); 7,34 ppm (m, 1 H); 7,22 ppm (t, 2H); 6,82 ppm (d, 1H); 4,75 ppm (t, 2H); 4,11 ppm (t, 2H); 4,01 ppm (m, 4H); 3,69 ppm (m, 4H); 3,43 ppm (s, 3H).

### Beispiel 6: Herstellung von 2-({2-[(2,4-Dihydroxy-1-naphthalenyl)-diazenyl]-phenyl}oxy)-N,N,N-trimethylethanaminium-chlorid

1,56 g (5 mmol) der Verbindung aus Stufe 1.1 werden in 20 ml Wasser und 1,85 g Salzsäure (32%ig) gelöst und unter Kühlung im Eisbad mit 0,42 g (6 mmol) Natriumnitrit in 3 ml Wasser diazotiert, wobei die Temperatur 5 °C nicht überschreiten sollte. Es wird noch 1 Stunde lang weitergerührt und sodann der Nitritüberschuß durch Zugabe von Sulfaminsäure zersetzt. Man gießt die Diazoniumsalzlösung unter Rühren in eine Lösung von 0,8 g (5 mmol) 1,3-Dihydroxynaphthalin in 30 ml Isopropanol. Durch Zugabe einer gesättigten Natriumcarbonatlösung stellt man den pH-Wert auf 9 bis 10 ein, wobei Kupplung eintritt und ein dicker Farbstoffbrei entsteht. Es wird noch 30 Minuten lang weitergerührt und abgesaugt. Das Rohprodukt wird anschließend in 30 ml 60 °C warmem Wasser gelöst und mit Salzsäure (32%ig) auf pH 1 eingestellt, wobei der Farbstoff als Chlorid ausfällt. Man läßt abkühlen und saugt erneut ab. Nach dem Trocknen erhält man 18 g (89 % der Theorie) oranges Produkt.
¹H-NMR (DMSO-*d₆*): δ = 10,09 (s, 1H); 9,48 (s, 1H); 8,40 (d, 1H); 8,00 (d, 1H); 7,93 (d, 1H); 7,62 (t, 1H); 7,49 (t, 1H); 7,20 (m, 3H); 6,20 (s, 1H); 4,66 (t, 2H); 3,93 (t, 2H); 3,32 ppm (s, 9H).

### Beispiel 7: Herstellung von 2-[(2-{[2-Hydroxy-7-(methyloxy)-1-naphthalenyl]-diazenyl}phenyl)oxy]-N,N,N-trimethylethanaminium-chlorid

Das Diazoniumsalz wird nach der in Beispiel 6 beschriebenen Weise hergestellt und dann auf 0,87 g (5 mmol) 7-Methoxy-2-naphthol gekuppelt.
Ausbeute: 1,1 g (77 % der Theorie) roter Farbstoff
¹H-NMR (DMSO-*d₆*): δ = 8,11 (d, 1H); 7,90 (d, 1H); 7,86 (d, 1H); 7,70 (d, 1H); 7,32 (m, 2H); 7,21 (t, 1H); 7,08 (dd, 1H); 6,65 (d, 1H); 4,71 (m, unstrukturiert, 2H); 3,95 (m, unstrukturiert, 2H); 3,33 (s, 9H); 3.31 ppm (s, 3H).

### Beispiel 8: Herstellung von 2-{[2-({2-Hydroxy-3-[(phenylamino)-carbonyl]-1-naphthalenyl}diazenyl)phenyl]oxy}-N,N,N-trimethyl-ethanaminium- chlorid

Das Diazoniumsalz wird nach der in Beispiel 6 beschriebenen Weise hergestellt und dann auf 1,32 g (5 mmol) 3-Hydroxy-2-naphthoesäure-anilid gekuppelt.
Ausbeute: 1,3 g (51 % der Theorie) rot-violetter Farbstoff
¹H-NMR (DMSO-*d₆*): δ = 8,89 (s, 1H); 8,53 (s, 1H); 8,19 (d, 1H); 8,01 (d, 1H); 7,92 (d, 1H); 7,86 (d, 1H); 7,74 (m, 3H); 7,52 (m, 1H); 7,38 (m, 4H); 7,15 (m, 2H); 4,77 (m, unstrukturiert, 2H); 4,07 (m, unstrukturiert, 2H);

### Beispiel 9: Herstellung von 2-[{4-[(2-Hydroxy-1-naphthalenyl)-diazenyl]-phenyl}-(methyl)amino]-N,N,N-trimethylethanaminium-methylsulfat

### Stufe 9.1: Herstellung von N,N,N'-Trimethyl-N'-(4-nitrophenyl)-1,2-ethandiamin

7,05 g (50 mmol) 4-Fluor-nitrobenzol und 8,17 g (80 mmol) N,N,N'-Trimethylethylendiamin werden in 50 ml Toluol gelöst, wobei ein leichter Temperaturanstieg zu verzeichnen ist. Nach Abklingen der Exothermie wird die Mischung 10 Stunden lang auf 80 °C erwärmt. Anschließend wird das Lösungsmittel am Rotationsverdampfer abgezogen und der ölige Rückstand in 80 ml 3molarer ethanolischer Salzsäure aufgenommen. Beim Rühren im Eisbad setzt allmählich Kristallisation ein. Nach 1 Stunde wird der entstandene Kristallbrei abgesaugt, mit wenig eiskaltem Ethanol nachgewaschen und im Vakuum bei 40 °C getrocknet.
Ausbeute: 11,0 g (85 % der Theorie) leuchtend gelbe Kristalle
¹H-NMR (DMSO-*d₆*): δ = 8,06 (d, 2H); 6,94 (d, 2H); 3,93 (t, 2H); 3,24 (t, 2H); 3,10 (s, 3H); 2,78 ppm (s, 6H).
Zur Freisetzung der Base werden 10 g des zuvor beschriebenen Produkts in 50 ml Wasser gelöst, durch Zugabe von gesättigter Natriumcarbonatlösung auf pH 10 eingestellt und dreimal mit je 25 ml Essigester extrahiert. Die vereinigten organischen Phasen werden über Magnesiumsulfat getrocknet und zur Trockne eingedampft.
Man erhält 8,0 g (93% der Theorie) eines gelben Öls
¹H-NMR (DMSO-*d₆*): δ = 8,02 (d, 2H); 6,74 (d, 2H); 3,55 (t, 2H); 3,06 (s, 3H); 2,40 (t, 2H); 2,18 ppm (s, 6H).

### Stufe 9.2: Herstellung von N,N,N-Trimethyl-2-[methyl(4-nitrophenyl)amino]-ethanaminium-methylsulfat

8,0 g (36 mmol) der Verbindung aus Stufe 9.1 werden in 50 ml Acetonitril gelöst und unter Rühren innerhalb von 5 Minuten tropfenweise mit 4,55 g (36 mmol) Dimethylsulfat versetzt, wobei sich die Reaktionsmischung leicht erwärmt und sich das Produkt allmählich kristallin abscheidet. Man rührt 3 Stunden bei Raumtemperatur weiter und saugt anschließend ab. Nachwaschen mit wenig Acetonitril und Trocknen bei 40 °C im Vakuum ergeben 11,0 g (88 % der Theorie) gelbe Kristalle.
¹H-NMR (DMSO-*d₆*): δ = 8,10 (d, 2H); 6,90 (d, 2H); 3,97 (t, 2H); 3,52 (t, 2H); 3,39 (s, 3H); 3,17 (s, 9H); 3,14 ppm (s, 3H).

### Stufe 9.3: Herstellung von 2-[(4-Aminophenyl)(methyl)amino]-N,N,N-trimethylethanaminium-methylsulfat

10 g (28,6 mmol) der Verbindung aus Stufe 9.2 werden in 80 ml Wasser gelöst und an 1 g Pd/C (10%ig) bei 9 bar Wasserstoffdruck hydriert. Nach 2 Stunden wird vom Katalysator abfiltriert und das Filtrat zur Trockne eingedampft. Man erhält 9,3 g (99,6 % der Theorie) eines braunen Öles.
¹H-NMR (DMSO-*d₆*): δ = 6,65 (d, 2H); 6,53 (d, 2H); 4,56 (s breit, 2H); 3,51 (m, unstrukturiert, 2H); 3,39 (s, 3H); 3,37 (m, unstrukturiert, 2H); 3,11 ppm (s, 9H).

### Stufe 9.4: Herstellung von 2-[{4-[(2-Hydroxy-1-naphthalenyl)-diazenyl]phenyl}-(methyl)amino]-N,N,N-trimethyl-ethanaminium-methylsulfat

1,6 g (5 mmol) der Verbindung aus Stufe 9.3 werden in 20 ml Wasser und 1,7 g Salzsäure (32%ig) gelöst und unter Rühren im Eisbad tropfenweise mit 0,35 g (5 mmol) Natriumnitrit in 2 ml Wasser versetzt. Man rührt im Eisbad 30 Minuten lang weiter, versetzt mit einigen Tropfen 10%iger Sulfaminsäurelösung und gießt in eine Lösung von 0,72 g (5 mmol) 2-Naphthol in 8 ml Isopropanol. Anschließend erhöht man den pH-Wert durch Zugabe von gesättigter Natriumcarbonatlösung auf 9, wobei sich eine braune Lösung bildet. Man neutralisiert nach 30 Minuten durch Zugabe von 2normaler Salzsäure und dampft zur Isolierung des Farbstoffs zunächst am Rotationsverdampfer zur Trockne ein. Der Rückstand wird dann in 80 ml Methanol aufgenommen, die anorganischen Salze werden abfiltriert und die Lösung sodann auf ca. 10 ml Gesamtvolumen aufkonzentriert. Durch Zugabe von ca. 30 ml Isopropanol fällt der Farbstoff aus.
Ausbeute: 1,5 g (63 % der Theorie) rotbraunes Produkt
¹H-NMR (DMSO-d₆): δ = 8,75 (d, 2H); 7,90 (m, 4H); 7,63 (t, 1H); 7,46 (t, 1 H); 7,17 (d, 1H); 6,98 (d, 2H); 3,97 (t, 2H); 3,57 (t, 2H); 3,38 (s, 3H); 3,21 (s, 9H); 3,14 ppm (s, 3H).

### Beispiel 10: Herstellung von 2-[{2-[(2-Hydroxy-1-naphthalenyl)-diazenyl]-phenyl}(methyl)amino]-N,N,N-trimethylethanaminium methylsulfat

### Stufe 10.1: Herstellung von N,N,N'-Trimethyl-N'-(2-nitrophenyl)-1,2-ethandiamin-hydrochlorid

7,05 g (50 mmol) 2-Fluor-nitrobenzol werden in 50 ml-Toluol gelöst. Zu dieser Lösung tropft man unter Rühren innerhalb von 5 Minuten 8,20 g (80 mmol) N,N,N'-Trimethylethylendiamin, wobei eine Erwärmung der Reaktionsmischung auf ca. 40 °C eintritt. Nach dem Abklingen der Exothermie wird der Ansatz 10 Stunden lang auf 80 °C erhitzt. Anschließend kühlt man ab und zieht am Rotationsverdampfer das Lösungsmittel ab. Man erhält einen öligen Rückstand, der mit 80 ml 3molarer ethanolischer Salzsäure versetzt und im Eisbad 1 Stunde lang gerührt wird. Dabei bildet sich allmählich ein oranger Niederschlag, der abgesaugt und mit wenig kaltem Ethanol nachgewaschen wird. Nach dem Trocknen im Vakuum bei 40 °C erhält man 7 g (69 % der Theorie) eines orangefarbenen Pulvers.
¹H-NMR (DMSO-*d₆*): δ = 10,90 (s breit, 1H); 7,81 (dd, 1H); 7,57 (m, 1H); 7,42 (dd, 1H); 7,08 (dt, 1H); 3,57 (t, 2H); 3,24 (t, 2H); 2,75 (s, 6H); 2,72 ppm (s, 3H).

### Stufe 10.2: Herstellung von N,N,N'-Trimethyl-N'-(2-nitrophenyl)-1,2-ethandiamin

6,50 g (25 mmol) der Verbindung aus Stufe 10.1 werden in in 50 ml Wasser gelöst und mit gesättigter Natriumcarbonatlösung alkalisch eingestellt. Anschließend extrahiert man dreimal mit je 20 ml Essigester, trocknet die vereinigten Phasen über Magnesiumsulfat, dampft ein und erhält 5,3 g (95 % der Theorie) eines gelben Öls.
¹H-NMR(DMSO-*d₆*): δ = 7,71 (dd, 1 H); 7,49 (t mit Feinaufspaltung, 1 H); 7,24 (dd, 1 H); 6,91 (t mit Feinaufspaltung, 1 H); 3,19 (t, 2H); 2,78 (s, 3H); 2,40 (t, 2H); 2,11 ppm (s, 6H).

### Stufe 10.3: Herstellung von N,N,N-Trimethyl-2-[methyl(2-nitrophenyl)amino]-ethanaminium methylsulfat

Man löst 5,0 g (22,4 mmol) des freien Amins aus Stufe 10.2 in 50 ml Acetonitril und gibt dazu unter Rühren innerhalb von 10 Minuten tropfenweise 2,83 g (22,4 mmol) Dimethylsulfat. Die Reaktionstemperatur steigt hierbei allmählich auf 45 °C an und es bildet sich ein Niederschlag. Nach 3stündigem Rühren filtriert man ab, wäscht mit wenig Acetonitril nach und trocknet bei 40 °C im Vakuum.
Ausbeute: 4,8 g (61 % der Theorie) gelbliches Pulver.
¹H-NMR (DMSO-*d₆*): δ = 7,82 (dd, 1H); 7,61 (t mit Feinaufspaltung, 1H); 7,44 (dd, 1H); 7,14 (t mit Feinaufspaltung, 1H); 3,58 (m, 4H); 3,38 (s, 3H); 3,12 (s, 9H); 2,76 ppm (s, 3H).

### Stufe 10.4: Herstellung von 2-[(2-Aminophenyl)(methyl)amino]-N,N,N-trimethylethan-aminium-methylsulfat

4,5 g (12,9 mmol) der Verbindung aus Stufe 10.3 werden in 80 ml Wasser gelöst und an 0,3 g Pd/C (10%ig) 2 Stunden lang bei 9 bar Wasserstoffdruck hydriert. Anschließend filtriert man den Katalysator ab und dampft das Filtrat zur Trockne ein. Man erhält 4,1 g (99 % der Theorie) eines violettstichigen Öls.
¹H-NMR (DMSO-*d₆*): δ = 7,00 (dd, 1H); 6,82 (t mit Feinaufspaltung; 1H); 6,70 (dd, 1H); 6,56 (t mit Feinaufspaltung, 1H); 4,83 (s, 2H); 3,44 (t, 2H); 3,39 (s, 3H); 3,26 (t, 2H); 3,09 (s, 9H); 2,57 ppm (s, 3H).

### Stufe 10.5: Herstellung von 2-[{2-[(2-Hydroxy-1-naphthalenyl)-diazenyl]phenyl}-(methyl)amino]-N,N,N-trimethylethan-aminium-methylsulfat

1,4 g (4,4 mmol) der Stufe 10.4 werden in 20 ml Wasser und 1,5 g Salzsäure (32%ig) gelöst, im Eisbad gekühlt und innerhalb von 10 Minuten mit 0,3 g (4,4 mmol) Natriumnitrit in 2 ml Wasser versetzt. Man rührt noch 30 Minuten weiter und gibt die Lösung anschließend zu einer Lösung aus 0,63 g (4,4 mmol) 2-Naphthol in 10 ml Isopropanol. Der pH-Wert wird durch Zugabe von gesättigter Natriumcarbonatlösung auf 8 bis 9 eingestellt, wobei Kupplung eintritt und eine braune Lösung erhalten wird. Nach 1 Stunde dampft man zur Trockene ein und versetzt zur Isolierung des reinen Farbstoffs den Rückstand mit 80 ml Methanol. Nach einstündigem Rühren bei Raumtemperatur werden die anorganischen Salze abfiltriert. Man engt das Filtrat auf ca. 20 % seines ursprünglichen Volumens ein und versetzt dann zur Kristallisation mit 30 ml Isopropanol. Nach Kühlen im Eisbad, Filtrieren und Trocknen im Vakuum bei 40 °C erhält man 1,6 g (67 % der Theorie) rotbraunes Produkt.
¹H-NMR (DMSO-*d*₆) δ = 8,48 (d, 1H); 8,10 (d mit Feinaufspaltung, 1H); 7,93 (d, 1H); 7,74 (d, 1H); 7,60 (t mit Feinaufspaltung, 1H); 7,46 (m, 2H); 7,35 (m, 2H); 6,78 (d, 1H); 3,55 (m, 4H); 3,38 (s, 3H); 3,14 (s, 9H); 2,78 ppm (s, 3H).

### Beispiel 11: Herstellung von 2-[{2-[(4-Hydroxy-1-naphthalenyl)-diazenyl]-phenyl}(methyl)amino]-N,N,N-trimethylethanaminium-methylsulfat

Man verfährt wie in Synthesebeispiel 9, Stufe 9.4 beschrieben, wobei anstatt des 2-Naphthols 0,63 g (4,4 mmol) 1-Naphthol verwendet werden. Ausbeute: 1,2 g (57 % der Theorie) rotbraunes Produkt
¹H-NMR (DMSO-*d*₆): δ = 8,71 (d, 1H); 8,13 (d, 1H); 7,89 (d, 1H); 7,58 (dd, 1H); 7,43 (t mit Feinaufspaltung, 1H); 7,22 (t, 1H); 7,12 (m, 2H); 7,01 (t mit Feinaufspaltung, 1H); 6,30 (d, 1H); 3,64 (m, 4H); 3,35 (s, 3H); 3,03 (s, 9H); 2,97 ppm (s, 3H).

### Beispiel 12: Herstellung von 2-({5-[(2-Hydroxy-1-naphthyl)diazenyl]-2-pyridinyl}oxy)-N,N,N-trimethylethanaminium-chlorid

### Stufe 12.1: Herstellung von N,N-Dimethyl-2-[(5-nitro-2-pyridinyl)oxy]-ethanamin

Man läßt die Mischung aus 7,93 g (50 mmol) 2-Chlor-5-nitro-pyridin, 4,9 g (55 mmol) 2,2-Dimethylamino-ethanol und 4 g (29 mmol) Kaliumcarbonat in 50 ml Dimethylformamid 3 Tage bei gelegentlichem Umschütteln bei Raumtemperatur stehen. Anschließend gießt man den Ansatz auf 200 ml Wasser und extrahiert dreimal mit je 30 ml Essigester. Nach dem Trocknen über Magnesiumsulfat und Verdampfen des Lösungsmittels bleibt ein gelbliches Öl zurück.
Ausbeute: 4,9 g (46 % der Theorie)
¹H-NMR(DMSO-d₆): δ = 9,07 (d, 1H); 8,46 (dd, 1H); 7,04 (d, 1H); 4,48 (t, 2H); 2,64 (t, 2H); 2,20 ppm (s, 6H).

### Stufe 12.2: Herstellung von N,N,N-Trimethyl-2-[(5-nitro-2-pyridinyl)-oxy]ethanaminium-methylsulfat

4,4 g (21 mmol) der Verbindung aus Stufe 12.1 werden in 50 ml Essigester gelöst und unter Rühren innerhalb von 5 Minuten mit 2,65 g (21 mmol) Dimethylsulfat versetzt. Unter leichter Exothermie bildet sich ein Niederschlag. Man rührt noch 1 Stunde lang weiter und saugt sodann ab. Nachwaschen mit Essigester und Trocknen bei 40 °C im Vakuum ergeben 6,5 g (92 % der Theorie) farblose Kristalle.
¹H-NMR (DMSO-*d₆*): δ = 9,13 (d, 1H); 8,56 (dd, 1H); 7,12 (d, 1H); 4,86 (m, 2H); 3,84 (m, 2H); 3,37 (s, 3H); 3,18 ppm (s, 9H).

### Stufe 12.3: Herstellung von 2-[(5-amino-2-pyridinyl)oxy]-N,N,N-trimethylethan-aminium-methylsulfat

4,0 g (12 mmol) der Verbindung aus Stufe 12.2 werden in 80 ml Wasser an 0,4 g Pd/C (10%ig) bei 9 bar Wasserstoffdruck hydriert. Nach 3 Stunden wird vom Katalysator abfiltriert und das Wasser am Rotationsverdampfer entfernt.
Ausbeute: 3,6 g (98,5 % der Theorie) violettstichiges Öl
¹H-NMR (DMSO-*d₆*): δ = 7,52 (d, 1H); 7,05 (dd, 1H); 6,61 (d, 1H); 4,87 (s, 2H); 4,56 (m, 2H); 3,71 (m, 2H); 3,38 (s, 3H); 3,15 ppm (s, 9H).

### Stufe 12.4: Herstellung von 2-({5-[(2-Hydroxy-1-naphthyl)diazenyl]-2-pyridinyl}oxy)-N,N,N-trimethylethanaminium-chlorid

1,81 g (5,9 mmol) der Verbindung aus Stufe 12.3 werden in 30 ml Wasser und 2,4 g Salzsäure (32%ig) gelöst und im Eisbad bei 0 bis 5 °C tropfenweise mit einer Lösung aus 0,41 g (6 mmol) Natriumnitrit versetzt. Man läßt noch 30 Minuten im Eisbad rühren und zersetzt dann den Nitritüberschuß durch Zugabe von Sulfaminsäure. Die erhaltene Diazoniumsalzlösung gibt man zu einer Lösung von 0,87 g (6 mmol) 2-Naphthol in 10 ml Isopropanol und stellt zur Kupplung den pH-Wert durch Zugabe von gesättigter Natriumcarbonatlösung auf 8 bis 9 ein. Man läßt noch 1 Stunde auskuppeln, neutralisiert mit 2normaler Salzsäure und saugt den Nierderschlag ab. Nach dem Abpressen des Filterkuchens und Trocknen im Vakuum bei 40 °C erhält man 1,7 g (74 % der Theorie) leuchtend oranges Produkt.
¹H-NMR (DMSO-*d₆*): δ = 14,55 (s breit, 1H); 8,91 (d, 1H); 8,75 (d, 1H); 8,47 (dd, 1H); 8,03 (d, 1H); 7,90 (d, 1H); 7,65 (t, 1H); 7,50 (t, 1H); 7,16 (d, 1H); 7,11 (d, 1H); 4,85 (m unstrukturiert, 2H); 3,86 (m, 2H); 3,22 ppm (s, 9H).

### Beispiel 13: Haarfärbemittel

Es wird eine Farbmasse der folgenden Zusammensetzung hergestellt:

| | |
|---|---|
| 4,0 g | Decylglucosid |
| 5,0 g | Ethanol |
| 0,0025 mol | Farbstoff gemäß Tabelle 1 |
| ad 100,0 g | Wasser, demineralisiert |

Der pH-Wert des Färbemittels wird mit Phosphorsäure oder Natronlauge auf 7 eingestellt. Anschließend wird das Mittel auf gebleichtes Haar aufgetragen und nach einer Einwirkzeit von 20 Minuten bei 40 °C mit Wasser ausgespült. Nach der Trocknung erhält man die in Tabelle 1 angegeben Färbungen.

**Tabelle 1:**

| **Farbstoff gemäß** | **L*** | **a*** | **b*** | **Farbe** |
|---|---|---|---|---|
| **Beispiel 1** | 44,35 | 54,77 | 45,12 | leuchtend orange-rot |
| **Beispiel 2** | 35,43 | 33,64 | 29,64 | rotbraun |
| **Beispiel 3** | 46,65 | 48,57 | 39,20 | leuchtend orange-rot |
| **Beispiel 4** | 48,71 | 31,52 | 58,47 | leuchtend orange |
| **Beispiel 5** | 51,65 | 49,73 | 43,40 | leuchtend orange-rot |
| **Beispiel 6** | 71,61 | 16,62 | 65,84 | goldgelb |
| **Beispiel 7** | 53,06 | 46,77 | 45,80 | orange-rot |
| **Beispiel 8** | 47,04 | 42,75 | 9,70 | rotviolett |
| **Beispiel 9** | 34,48 | 21,39 | 16,72 | braun |
| **Beispiel 10** | 33,04 | 22,09 | 16,34 | bordeauxrot |
| **Beispiel 11** | 35,60 | 10,28 | 21,48 | kastanienbraun |
| **Beispiel 12** | 55,27 | 47,39 | 63,62 | brillantorange |

### Beispiele 14 bis 18: Haarfärbemittel

Es wird eine Farbmasse der folgenden Zusammensetzung hergestellt:

| | |
|---|---|
| 10,00 g | Ethanol |
| 1,00 g | Hydroxyethylcellulose |
| 0,29 g | Farbstoff gemäß Beispiel 1 |
| X g | direktziehende Farbstoffe gemäß Tabelle 2 |
| ad 100,00 g | Wasser, demineralisiert |

Das Färbemittel wird auf gebleichtes Haar aufgetragen und nach einer Einwirkzeit von 20 Minuten bei 40 °C mit Wasser ausgespült.
Anschließend wird das Haar getrocknet.

**Tabelle 2:**

| **Beispiel Nr.** | **direktziehende Farbstoffe** | **Farbe** |
|---|---|---|
| **14** | 0,25 g Basic Yellow 57 | leuchtend orange |
| **15** | 0,18 g Basic Red 76 | orange |
| **16** | 0,35 g Basic Brown 17 | rotbraun |
| **17** | 0,42 g Basic Blue 99 | auberginefarben |
| **18** | 0,23 g Basic Yellow 57 | anthrazit |
| | 0,31 g Basic Brown 17 | |
| | 0,28 g Basic Blue 99 | |

Die angegebenen L*a*b*-Farbmesswerte wurden mit einem Farbmessgerät der Firma Minolta, Typ Chromameter II, ermittelt. Hierbei steht der L*-Wert für die Helligkeit (das heißt je geringer der L*-Wert ist, umso größer ist die Farbintensität), während der a*-Wert ein Maß für den Rotanteil ist (das heißt je größer der a*-Wert ist, umso größer ist der Rotanteil), und der b*-Wert ein Maß für den Blauanteil der Farbe ist (das heißt je negativer der b*-Wert ist, umso größer ist der Blauanteil).

Alle Prozentangaben stellen -soweit nicht anders angegeben-Gewichtsprozente dar.

## Patentansprüche

1. Kationische Naphthyldiazofarbstoffe der allgemeinen Formel (I), 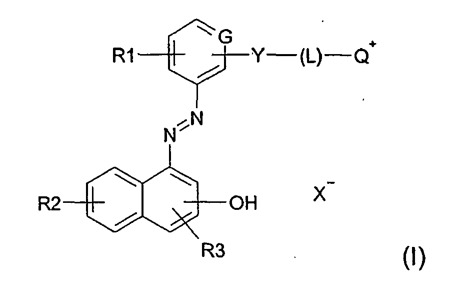 worin
**R1** ein Wasserstoffatom, ein Halogenatom, eine geradkettige oder verzweigte (C1-C4)-Alkylgruppe, eine geradkettige oder verzweigte (C1-C4)-Alkoxygruppe, eine Phenylgruppe oder eine (C2-C4)-Hydroxyalkylgruppe darstellt;
R2 und R3 gleich oder verschieden sein können und unabhängig voneinander ein Wasserstoffatom, eine Hydroxygruppe, eine Aminogruppe, eine Acetylaminogruppe, eine (C1-C6)-Alkoxygruppe, eine (C2-C4)-Hydroxyalkoxygruppe, eine (C3-C6)-Di- oder Polyhydroxyalkoxygruppe, eine -COOR-Gruppe , eine -NRR'-Gruppe oder eine -CONRR'-Gruppe darstellen, wobei **R** und **R'** gleich oder verschieden sein können und ein Wasserstoffatom, eine geradkettige oder verzweigte (C1-C6)-Alkylgruppe oder eine Hydroxyethylgruppe darstellen, beziehungsweise **R** und **R'** gemeinsam mit dem Stickstoffatom, an das sie gebunden sind, einen Heterozyklus mit mindestens vier Ringgliedern bilden, der gegebenenfalls weitere Heteroatome enthalten kann, wobei R und R' sowie der zuvor beschriebene Heterozyklus mit einer Alkylgruppe, einer Alkoxygruppe, einer Hydroxyalkylgruppe oder einer Aminoalkylgruppe substituiert sein können;
G ein Stickstoffatom oder eine Methingruppe (CH) darstellt;
Y ein Sauerstoffatom oder eine N-(C1-C4)-Alkylgruppe darstellt;
L eine Brückengruppe darstellt und für eine geradkettige oder verzweigte (C1-C14)-Alkylengruppe, die gegebenenfalls durch ein oder mehrere Heteroatome unterbrochen sein kann, steht, wobei die Brückengruppe gegebenenfalls mit einer oder mehreren Hydroxygruppen, Monohydroxy-(C2-C6)-alkylgruppen, Polyhydroxy-(C2-C6)-alkylgruppen oder (C1-C6)-Alkoxygruppen substituiert ist;
**Q⁺** steht für eine gesättigte kationische Gruppe der Formel (II) oder eine ungesättigte kationische Gruppe der Formeln (III) bis (V), 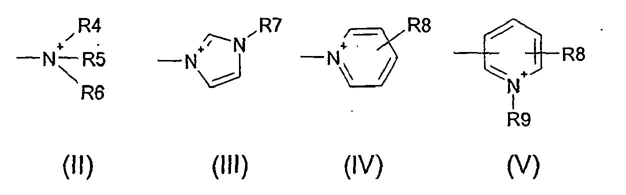 in denen
R4 bis R6 gleich oder verschieden sein können und unabhängig voneinander eine geradkettige oder verzweigte (C1-C6)-Alkylgruppe eine (C2-C4)-Hydroxyalkylgruppe, eine (C3-C6)-Dihydroxyalkylgruppe, eine (C3-C6)-Polyhydroxyalkylgruppe oder eine (C1-C6)-Alkoxy-(C1-C4)-alkylgruppe darstellen, wobei zwei der Gruppen R4 bis R6 zusammen mit dem Stickstoffatom, an das sie gebunden sind, einen fünf- oder sechsgliedrigen Heterozyklus bilden, der gegebenenfalls durch ein oder mehrere Heteroatome wie ein Sauerstoffatom, ein Schwefelatom oder ein Stickstoffatom unterbrochen sein kann und gegebenenfalls weitere Substituenten, wie beispielsweise ein Halogenatom, eine Hydroxygruppe, eine Aminogruppe, eine geradkettige oder verzweigte (C1-C6)-Alkylgruppe, eine (C1-C6)-Alkoxygruppe, eine (C1-C6)-Alkoxy-(C1-C4)-alkylgruppe oder eine Hydroxyethylgruppe aufweisen kann;
R7 eine geradkettige oder verzweigte (C1-C8)-Alkylgruppe, eine Allylgruppe, eine Vinylgruppe, eine Hydroxyethylgruppe oder eine Benzylgruppe darstellt;
**R8** ein Wasserstoffatom, eine geradkettige oder verzweigte (C1-C9)-Alkylgruppe, eine Aminogruppe, eine Di-(C1-C6)-alkylaminogruppe oder eine Pyrrolidinogruppe darstellt;
**R9** eine geradkettige oder verzweigte (C1-C8)-Alkylgruppe, eine Allylgruppe, eine Vinylgruppe, eine Hydroxyethylgruppe, eine Dihydroxypropylgruppe oder eine Benzylgruppe darstellt; und
X⁻ für ein Anion steht.

2. Farbstoffe der Formel (I) gemäß Anspruch 1, **dadurch gekennzeichnet, dass**
R1 ein Wasserstoffatom, ein Chloratom oder eine Methylgruppe darstellt,
R2 und R3 gleich oder verschieden sind und unabhängig voneinander Wasserstoff, eine Hydroxygruppe, eine Methoxygruppe, eine -NRR'-Gruppe oder eine -CONRR'-Gruppe darstellen, wobei **R** und **R'** gleich oder verschieden sein können und ein Wasserstoffatom, eine Methylgruppe oder eine Hydroxyethylgruppe darstellen, oder aber **R** und **R'** gemeinsam mit dem Stickstoffatom, an das sie gebunden sind, einen Heterozyklus mit fünf oder sechs Ringgliedern bilden;
**G** für ein Stickstoffatom oder eine Methingruppe (CH) steht;
**Y** gleich Sauerstoff oder einer N-Methylgruppe ist;
**L** für einegeradkettige (C2-C4)-Brückengruppe steht;
**Q⁺** eine gesättigte kationische Gruppe der Formel (II) oder eine ungesättigte kationische Gruppe der Formel (III) bis (V) darstellt, wobei die Restgruppen **R4** bis **R6** gleich oder verschieden sein können und unabhängig voneinander eine geradkettige (C1-C3)-Alkylgruppe, eine Hydroxyethylgruppe oder eine Methoxyethylgruppe darstellen, oder aber zwei der Gruppen R4 bis R6 gemeinsam mit dem Stickstoffatom an das sie gebunden sind einen fünfgliedrigen oder sechsgliedrigen Heterozyklus bilden;
**R7** eine Methylgruppe oder eine Hydroxyethylgruppe darstellt;
**R8** ein Wasserstoffatom, eine Methylgruppe, eine Dimethylaminogruppe oder eine Pyrrolidinogruppe darstellt;
R9 gleich einer Methylgruppe, einer Ethylgruppe oder einer Hydroxyethylgruppe ist und
X⁻ gleich einem Chloridanion, einem Bromidanion oder einem Methylsulfatanion ist.

3. Farbstoffe der Formel (I) gemäß Anspruch 1 oder 2, **dadurch gekennzeichnet, dass** sie ausgewählt sind aus 2-{2-[(2-Hydroxy-1-naphthyl)diazenyl]phenoxy}-N,N,N-trimethyl-ethanaminium-methylsulfat, 2-{2-[(4-Hydroxy-1-naphthyl)diazenyl]phenoxy}-N,N,N-trimethyl-ethanaminium-chlorid, 2-(2-{2-[(2-Hydroxy-1-naphthyl)diazenyl]phenoxy}-ethyl)-1-methylpyridinium-methylsulfat, 2-{2-[(2,7-Dihydroxy-1-naphthyl)-diazenyl]phenoxy}-N,N,N-trimethyl-ethanaminium-chlorid, 4-(2-{2-[(2-Hydroxy-1-naphthyl)diazenyl]phenoxy}ethyl)-4-methylmorpholin-4-ium-chlorid, 2-[(2-{[2-Hydroxy-7-(methyloxy)-1-naphthalenyl]diazenyl}phenyl)oxy]-N,N,N-trimethylethanaminium-chlorid, 2-[{4-[(2-Hydroxy-1-naphthalenyl)diazenyl]phenyl}-(methyl)amino]-N,N,N-trimethylethanaminium-methylsulfat, 2-[{2-[(2-Hydroxy-1-naphthalenyl)-diazenyl]-phenyl}(methyl)amino]-N,N,N-trimethylethanaminium-methylsulfat, 2-[{2-[(4-Hydroxy-1-naphthalenyl)diazenyl]-phenyl}(methyl)-amino]-N,N,N-trimethylethanaminium-methylsulfat, 2-({5-[(2-Hydroxy-1-naphthyl)diazenyl]-2-pyridinyl}oxy)-N,N,N-trimethylethanaminium-chlorid, 2-({3-[(2-Hydroxy-1-naphthyl)diazenyl]-2-pyridinyl}oxy)-N,N,N-trimethyl-ethanaminium-chlorid, 2-({3-[(4-Hydroxy-1-naphthyl)diazenyl]-2-pyridinyl}oxy)-N,N,N-trimethylethanaminium-chlorid, 2-{3-[(2-Hydroxy-1-naphthyl)diazenyl]phenoxy}-N,N,N-trimethylethanaminium-chlorid, 3-(2-{2-[(2-Hydroxy-1-naphthyl)diazenyl]phenoxy}ethyl)-1-methyl-1 H-imidazol-3-ium chlorid, 2-({2-[(2,4-Dihydroxy-1-naphthalenyl)-diazenyl]-phenyl}oxy)-N,N,N-trimethylethanaminium-chlorid und 2-{[2-({2-Hydroxy-3-[(phenylamino)-carbonyl]-1-naphthalenyl}diazenyl)phenyl]oxy}-N,N,N-trimethyl-ethanaminium-chlorid.

4. Mittel zur Färbung von Keratinfasern, **dadurch gekennzeichnet, dass** es mindestens einen Farbstoff der Formel (I) nach einem der Ansprüche 1 bis 3 enthält.

5. Mittel nach Anspruch 4, **dadurch gekennzeichnet, dass** es den Farbstoff der Formel (I) in einer Gesamtmenge von 0,01 bis 10 Gewichtsprozent enthält.

6. Mittel nach Anspruch 4 oder 5, **dadurch gekennzeichnet, dass** es neben den Farbstoffen der Formel (I) weitere Farbstoffe enthält.

7. Mittel nach Anspruch 6, **dadurch gekennzeichnet, dass** der weitere Farbstoff ausgwählt ist aus 3-((4,5-Dihydro-3-methyl-5-oxo-1-phenyl-1 H-pyrazol-4-yl)azo)-N,N,N-trimethyl-benzen-aminiumchlorid, 3-[(3-Methyl-5-hydroxy-1-phenyl-1H-pyrazol-4-yl)azo]-trimethylammonio-benzol-chlorid, 8-[(4-Aminophenyl)azo]-7-hydroxy-N,N,N-trimethyl-2-naphthalinaminiumchlorid, 8-[(4-Amino-3-nitrophenyl)azo]-7-hydroxy-N,N,N-trimethyl-2-naphthalin-aminium-chlorid, 8-[(4-Amino-2-nitrophenyl)-azo]-7-hydroxy-N,N,N-trimethyl-2-naphthalinaminium-chlorid, 7-Hydroxy-N,N,N-trimethyl-8-{[2-(methyloxy)phenyl]-azo}-2-naphthalinaminium-chlorid, 3-((4-Amino-6-bromo-5,8-dihydro-1-hydroxy-8-imino-5-oxo-2-naphthalenyl)amino)-N,N,N-trimethyl-benzolamoniumchlorid und N,N-Dimethyl-3-{[4-(methylamino)-9,10-dioxo-9,10-dihydro-1-anthracenyl]-amino}-N-propyl-1-propanaminium-bromid.

8. Mittel nach Anspruch 6 oder 7, **dadurch gekennzeichnet, dass** es die weiteren Farbstoffe in einer Gesamtmenge von 0,01 bis 15 Gewichtsprozent enthält.

9. Mittel nach einem der Ansprüche 4 bis 8, **dadurch gekennzeichnet, dass** es mindestens ein natürliches oder synthetisches Polymer beziehungsweise modifiziertes Polymer natürlichen Ursprungs enthält und in Form eines Tönungsfestigers oder Farbfestigers vorliegt.

10. Mittel nach einem der Ansprüche 4 bis 9, **dadurch gekennzeichnet, dass** es ein Haarfärbemittel ist.

## Claims

1. Cationic naphthyldiazo dyes of the general formula (I), 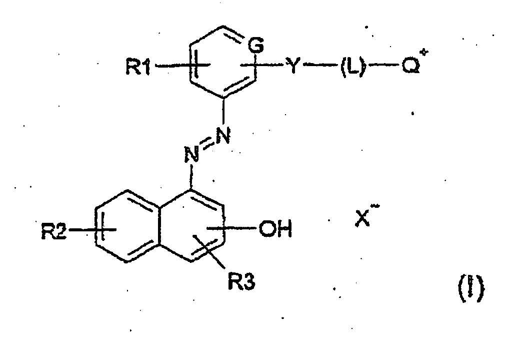 in which
**R1** is a hydrogen atom, a halogen atom, a straight-chain or branched (C1-C4)-alkyl group, a straight-chain or branched (C1-C4)-alkoxy group, a phenyl group or a (C2-C4)-hydroxyalkyl group;
R2 and R3 may be identical or different and, independently of one another, are a hydrogen atom, a hydroxy group, an amino group, an acetylamino group, a (C1-C6)-alkoxy group, a (C2-C4)-hydroxyalkoxy group, a (C3-C6)-di- or polyhydroxyalkoxy group; a -COOR group, a -NRR' group or a -CONRR' group, where **R** and **R'** may be identical or different and are a hydrogen atom, a straight-chain or branched (C1-C6)-alkyl group or a hydroxyethyl group, or R and R', together with the nitrogen atom to which they are bonded, form a heterocycle with at least four ring members, which can optionally contain further heteroatoms, where R and R', and the heterocycle described above may be substituted by an alkyl group, an alkoxy group, a hydroxyalkyl group or an aminoalkyl group;
G is a nitrogen atom or a methine group (CH);
Y is an oxygen atom or a N-(C1-C4)-alkyl group;
L is a bridge group and is a straight-chain or branched (C1-C14)-alkylene group, which may optionally be interrupted by one or more heteroatoms, where the bridge group is optionally substituted by one or more hydroxy groups, monohydroxy-(C2-C6)-alkyl groups, polhydroxy-(C2-C6)-alkyl groups or (C1-C6)-alkoxy groups;
Q⁺ is a saturated cationic group of the Formula (II) or an unsaturated cationic group of the Formulae (III) to (V), 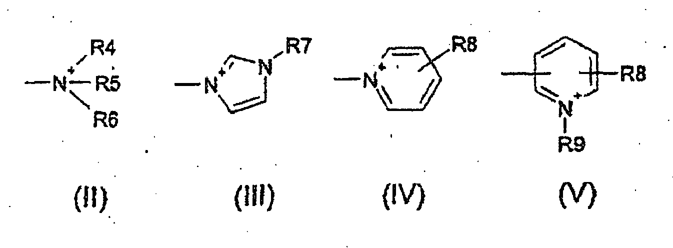 in which
**R4** to **R6** may be identical or different and, independently of one another, are a straight-chain or branched (C1-C6)-alkyl group, a (C2-C4)-hydroxyalkyl group, a (C3-C6)-dihydroxyalkyl group, a (C3-C6)-polyhydroxyalkyl group or a (C1-C6)-alkoxy-(C1-C4)-alkyl group, where two of the groups R4 to R6, together with the nitrogen atom to which they are bonded, form a five- or six-membered heterocycle, which may optionally be interrupted by one or more heteroatoms, such as an oxygen atom, a sulphur atom or a nitrogen atom, and can optionally have further substituents, such as, for example, a halogen atom, a hydroxy group, an amino group, a straight-chain or branched (C1-C6)-alkyl group, a (C1-C6)-alkoxy group, a (C1-C6)-alkoxy-(C1-C4)-alkyl group or a hydroxyethyl group;
**R7** is a straight-chain or branched (C1-C8)-alkyl group, an allyl group, a vinyl group, a hydroxyethyl group or a benzyl group;
**R8** is a hydrogen atom, a straight-chain or branched (C1-C9)-alkyl group, an amino group, a di(C1-C6)-alkylamino group or a pyrrolidino group;
**R9** is a straight-chain or branched (C1-C8)-alkyl group, an allyl group, a vinyl group, a hydroxyethyl group, a dihydroxypropyl group or a benzyl group; and
**x⁻** is an anion.

2. Dyes of the Formula (I) according to Claim 1, **characterized in that**
**R1** is a hydrogen atom, a chlorine atom or a methyl group,
R2 and R3 are identical or different and, independently of one another, are hydrogen, a hydroxy group, a methoxy group, a -NRR' group or a -CONRR' group, where **R** and **R'** may be identical or different and are a hydrogen atom, a methyl group or a hydroxyethyl group, or else **R** and **R**', together with the nitrogen atom to which they are bonded, form a heterocycle with five or six ring members;
G is a nitrogen atom or a methine group (CH);
Y is oxygen or a N-methyl group;
L is a straight-chain (C2-C4) bridge group;
Q⁺ is a saturated cationic group of the Formula (II) or an unsaturated cationic group of the Formula (III) to (V), where the radical groups R4 to R6 may be identical or different and, independently of one another, are a straight-chain (C1-C3) -alkyl group, a hydroxyethyl group or a methoxyethyl group, or else two of the groups R4 to R6, together with the nitrogen atom to which they are bonded, form a five-membered or six-membered heterocycle;
R7, is a methyl group or a hydroxyethyl group;
R8 is a hydrogen atom, a methyl group, a dimethylamino group or a pyrrolidino group;
R9 is a methyl group, an ethyl group or a hydroxyethyl group and
X⁻ is a chloride anion, a bromide anion or a methylsulphate anion.

3. Dyes of the Formula (I) according to Claim 1 or 2, **characterized in that** they are selected from 2-{2-[(2-hydroxy-1-naphthyl)diazenyl]phenoxy}-N,N,N-trimethylethaneaminium methylsulphate, 2-{2-[(4-hydroxy-1-naphthyl)diazenyl]phenoxy}-N,N,N-trimethylethaneaminium chloride, 2-(2-{2-[(2-hydroxy-1-naphthyl,diazenyl]phenoxy}ethyl)-1-methylpyridinium methylsulphate, 2-{2-[(2,7-dihydroxy-1-naphthyl)diazenyl]phenoxy}-N,N,N-trimethylethaneaminium chloride, 4-(2-{2-[(2-hydroxy-1-naphthyl)diazenyl]phenoxy}ethyl)-4-methyl-morpholin-4-ium chloride, 2-[(2-{[2-hydroxy-7-(methyloxy)-1-naphthalenyl]diazenyl}phenyl)oxy]-N,N,N-trimethylethaneaminium chloride, 2-[{4-[(2-hydroxy-1-naphthalenyl)diazenyl]phenyl}-(methyl)amino]-N,N,N-trimethylethaneaminium methylsulphate, 2-[{2-[(2-hydroxy-1-naphthalenyl)diazenyl]phenyl}(methyl)amino]]-N,N,N-trimethylethaneaminium methylsulphate, 2-[{2-[(4-hydroxy-1-naphthalenyl)diazenyl]phenyl}(methyl)amino]-N,N,N-trimethylethaneaminium methylsulphate, 2-({5-[(2-hydroxy-1-naphthyl)diazenyl]-2-pyridinyl}oxy)-N,N,N-trimethylethaneaminium chloride, 2-({3-[(2-hydroxy-1-naphthyl)diazenyl]-2-pyridinyl}oxy)-N,N,N-trimethylethaneaminium chloride, 2-({3-[(4-hydroxy-1-naphthyl)diazenyl]-2-pyridinyl}oxy)-N,N,N-trimethylethaneaminium chloride, 2-{3-[(2-hydroxy-1-naphthyl)diazenyl]phenoxy}-N,N,N-trimethylethaneaminium chloride, 3(2-{2-[(2-hydroxy-1-naphthyl)diazenyl]phenoxy}ethyl)-1-methyl-1*H*-imidazol-3-ium chloride, 2-({2-[(2,4-dihydroxy-1-naphthalenyl)diazenyl]-phenyl}oxy)-N,N,N-trimethylethaneaminium chloride and 2-{[2-({2-hydroxy-3-[(phenylamino)carbonyl]-1-naphthalenyl}diazenyl)phenyl]oxy}-N,N,N-trimethylethaneaminium chloride.

4. Agent for colouring keratin fibres, **characterized in that** it comprises at least one dye of the Formula (I) according to one of Claims 1 to 3.

5. Agent according to Claim 4, **characterized in that** it comprises the dye of the Formula (I) in a total amount of from 0.01 to 10% by weight.

6. Agent according to Claim 4 or 5, **characterized in that**, besides the dyes of the Formula (I), it comprises further dyes.

7. Agent according to Claim 6, **characterized in that** the further dye is selected from 3-((4,5-dihydro-3-methyl-5-oxo-1-phenyl-1H-pyrazol-4-yl)azo)-N,N,N-trimethylbenzeneaminium chloride, 3-[(3-methyl-5-hydroxy-1-phenyl-1H-pyrazol-4-yl)azo]-trimethylammoniobenzene chloride, 8-[(4-aminophenyl)azo]-7-hydroxy-N,N,N-trimethyl-2-naphthaleneaminium chloride, 8-[(4-amino-3-nitrophenyl)azo]-7-hydroxy-N,N,N-trimethyl-2-naphthaleneaminium chloride, 8-[(4-amino-2-nitrophenyl)azo]-7-hydroxy-N,N,N-trimethyl-2-naphthaleneaminium chloride, 7-hydroxy-N,N,N-trimethyl-8-{[2-(methyloxy)phenyl]azo)-2-naphthaleneaminium chloride, 3-((4-amino-6-bromo-5,8-dihydro-1-hydroxy-8-imino-5-oxo-2-naphthalenyl)amino)-N,N,N-trimethylbenzeneaminium chloride and N,N-dimethyl-3-{[4-(methylamino)-9,10-dioxo-9,10-dihydro-1-anthracenyl]-amino}-N-propyl-1-propaneaminium bromide.

8. Agent according to Claim 6 or 7, **characterized in that** it comprises the further dyes in a total amount of from 0.01 to 15% by weight.

9. Agent according to one of Claims 4 to 8, **characterized in that** it comprises at least one natural or synthetic polymer or modified polymer of natural origin and is in the form of a tint setting composition or colour setting composition.

10. Agent according to one of Claims 4 to 9, **characterized in that** it is a hair colorant.

## Revendications

1. Colorants naphtyldiazo de formule générale (I), 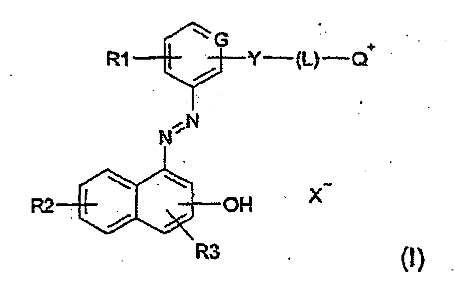 dans laquelle
**R1** représente un atome d'hydrogène, un atome d'halogène, un groupe alkyle en C₁-C₄ à chaîne droite ou ramifiée, un groupe alcoxy en C₁-C₄ à chaîne droite ou ramifiée, un groupe phényle ou un groupe hydroxyalkyle en C₂-C₄ ;
**R2** et **R3** peuvent être identiques ou différents et représentent, indépendamment l'un de l'autre, un atome d'hydrogène, un groupe hydroxy, un groupe amino, un groupe acétylamino, un groupe alcoxy en C₁-C₆, un groupe hydroxyalcoxy en C₂-C₄, un groupe di- ou polyhydroxyalcoxy en C₃-C₆, un groupe -COOR, un groupe -NRR' ou un groupe -CONRR', **R** et **R'** pouvant être identiques ou différents et représentant un atome d'hydrogène, un groupe alkyle en C₁-C₆ à chaîne droite ou ramifiée ou un groupe hydroxyéthyle, ou **R** et **R'** formant ensemble, avec l'atome d'azote auquel ils sont fixés, un hétérocycle comportant au moins quatre chaînons formant le cycle, qui peut éventuellement contenir d'autres hétéroatomes, R et R' ainsi que l'hétérocycle décrit précédemment pouvant être substitués par un groupe alkyle, un groupe alcoxy, un groupe hydroxyalkyle ou un groupe aminoalkyle ;
**G** représente un atome d'azote ou un groupe méthine (CH) ;
**Y** représente un atome d'oxygène ou un groupe N-alkyle(C₁-C₄) ;
**L** est un groupe pontant et représente un groupe alkylène en C₁-C₁₄ à chaîne droite ou ramifiée, qui peut éventuellement être interrompu par un ou plusieurs hétéroatomes, le groupe pontant étant éventuellement substitué par un ou plusieurs groupes hydroxy, monohydroxyalkyle en C₂-C₆, polyhydroxyalkyle en C₂-C₆ ou alcoxy en C₁-C₆ ;
**Q⁺** représente un groupe cationique saturé de formule (II) ou un groupe cationique insaturé de formules (III) à (V), 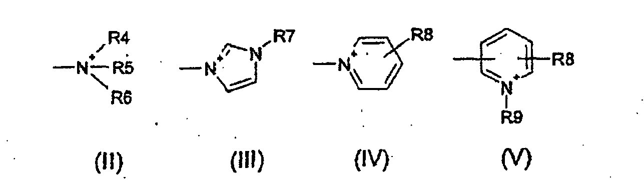 dans lesquelles
**R4** à **R6** peuvent être identiques ou différents et représentent chacun indépendamment un groupe alkyle en C₁-C₆ à chaîne droite ou ramifiée, un groupe hydroxyalkyle en C₂-C₄, un groupe dihydroxyalkyle en C₃-C₆, un groupe polyhydroxyalkyle en C₃-C₆ ou un groupe alcoxy(C₁-C₆)-alkyle(C₁-C₄), deux des groupes R4 à R6 formant ensemble, avec l'atome d'azote auquel ils sont fixés, un hétérocycle à cinq ou six chaînons, qui peut éventuellement être interrompu par un ou plusieurs hétéroatomes tels qu'un atome d'oxygène, un atome de soufre ou un atome d'azote et peut éventuellement comporter d'autres substituants, comme par exemple un atome d'halogène, un groupe hydroxy, un groupe amino, un groupe alkyle en C₁-C₆ à chaîne droite ou ramifiée, un groupe alcoxy en C₁-C₆, un groupe alcoxy(C₁-C₆)-alkyle(C₁-C₄) ou un groupe hydroxyéthyle ;
**R7** représente un groupe alkyle en C₁-C₈ à chaîne droite ou ramifiée, un groupe allyle, un groupe vinyle, un groupe hydroxyéthyle ou un groupe benzyle ;
**R8** représente un atome d'hydrogène, un groupe alkyle en C₁-C₉ à chaîne droite ou ramifiée, un groupe amino, un groupe dialkyl(C₁-C₆)amino ou un groupe pyrrolidino ;
**R9** représente un groupe alkyle en C₁-C₈ à chaîne droite ou ramifiée, un groupe allyle, un groupe vinyle, un groupe hydroxyéthyle, un groupe dihydroxypropyle ou un groupe benzyle ; et
**X⁻** représente un anion.

2. Colorants de formule (I) selon la revendication-1, **caractérisé en ce que**
**R1** représente un atome d'hydrogène, un atome de chlore ou un groupe méthyle ;
**R2** et **R3** peuvent être identiques ou différents et représentent, indépendamment l'un de l'autre, un atome d'hydrogène, un groupe hydroxy, un groupe méthoxy, un groupe-NRR' ou un groupe -CONRR', **R** et **R'** pouvant être identiques ou différents et représentant un atome d'hydrogène, un groupe méthyle ou un groupe hydroxyéthyle, ou bien **R** et **R'** formant ensemble, avec l'atome d'azote auquel ils sont fixés, un hétérocycle à cinq ou six chaînons formant le cycle ;
**G** représente un atome d'azote ou un groupe méthine (CH) ;
**Y** représente un atome d'oxygène ou un groupe N-méthyle ;
**L** représente un groupe pontant en C₂-C₄ à chaîne droite ;
**Q⁺** représente un groupe cationique saturé de formule (II) ou un groupe cationique insaturé de formules (III) à (V), les groupes restants R4 à R6 pouvant être identiques ou différents et représentant chacun indépendamment un groupe alkyle en C₁-C₃ à chaîne droite ou ramifiée, un groupe hydroxyéthyle ou un groupe méthoxyéthyle, ou bien deux des groupes R4 à R6 formant ensemble, avec l'atome d'azote auquel ils sont fixés, un hétérocycle à cinq ou six chaînons ;
**R7** représente un groupe méthyle ou un groupe hydroxyéthyle ;
**R8** représente un atome d'hydrogène, un groupe méthyle, un groupe diméthylamino ou un groupe pyrrolidino ;
**R9** représente un groupe méthyle, un groupe éthyle ou un groupe hydroxyéthyle ; et
**X⁻** représente un anion chlorure, un anion bromure ou un anion méthylsulfate.

3. Colorants de formule (I) selon la revendication 1 ou 2, **caractérisés en ce que** qu'il sont choisis parmi le méthylsulfate de 2-{2-[(2-hydroxy-1-naphtyl)diazényl]phénoxy}-N,N,N-triméthyl-éthanaminium, le chlorure de 2-{2-[(4-hydroxy-1-naphtyl)diazényl]phénoxy}-N,N,N-triméthyl-éthanaminium, le méthylsulfate de 2-(2-{2-[(2-hydroxy-1-naphtyl)diazényl]phénoxy}-éthyl)-1-méthylpyridinium, le chlorure de 2-{2-[(2,7-dihydroxy-1-naphtyl)diazényl]phénoxy}-N,N,N-triméthyl-éthanaminium, le chlorure de 4-(2-{2-[(2-hydroxy-1-naphtyl)diazényl]phénoxy}éthyl)-4-méthyl-morpholin-4-ium, le chlorure de 2-[(2-{[2-hydroxy-7-(méthyloxy)-1-naphtalényl]diazényl}-phényl)oxy]-N,N,N-triméthyl-éthanaminium, le méthylsulfate de 2-[{4-[(2-hydroxy-1-naphtalényl)-diazényl]phényl}-(méthyl)amino]-N,N,N-triméthyl-éthanaminium, le méthylsulfate de 2-[{2-[(2-hydroxy-1-naphtalényl)diazényl]phényl}(méthyl)-amino]-N,N,N-triméthyl-éthanaminium, le méthylsulfate de 2-[{2-[(4-hydroxy-1-naphtalényl)-diazényl]phényl}(méthyl)amino]-N,N,N-triméthyl-, éthanaminium, le chlorure de 2-({5-[(2-hydroxy-1-naphtyl)diazényl]-2-pyridinyl}oxy)-N,N,N-triméthyl-éthanaminium, le chlorure de 2-({3-[(2-hydroxy-1-naphtyl)diazényl]-2-pyridinyl}oxy)-N,N,N-triméthyl-éthanaminium, le chlorure de 2-({3-[(4-hydroxy-1-naphtyl)diazényl]-2-pyridinyl}-oxy)-N,N,N-triméthyl-éthanaminium, le chlorure de 2-{3-[(2-hydroxy-1-naphtyl)diazényl]phénoxy}-N,N,N-trimethyléthanaminium, le chlorure de 3-(2-{2-[(2-hydroxy-1-naphtyl)diazényl]phénoxy}éthyl)-1-méthyl-1H-imidazol-3-ium, le chlorure de 2-({2-[(2,4-dihydroxy-1-naphthalényl)-diazényl]phényl}-oxy)-N,N,N-triméthyléthanaminium et le chlorure de 2-{[2-({2-hydroxy-3-[(phénylamino)-carbonyl]-1-naphthalényl}diazényl)phényl]oxy}-N,N,N-triméthylethanaminium.

4. Composition pour la teinture de fibres de kératine, **caractérisée en ce qu'**elle contient au moins un colorant de formule (I) selon l'une quelconque des revendications 1 à 3.

5. Composition selon la revendication 4, **caractérisée en ce qu'**elle contient le colorant de formule (I) en une quantité totale de 0,01 à 10 % en poids.

6. Composition selon la revendication 4 ou 5, **caractérisée en ce qu'**en plus des colorants de formule (I) elle contient d'autres colorants.

7. Composition selon la revendication 6, **caractérisée en ce que** l'autre colorant est choisi parmi le chlorure de 3-((4,5-dihydro-3-méthyl-5-oxo-1-phenyl-1H-pyrazol-4-yl)azo)-N,N,N-triméthylbenzène-aminium, le chlorure de 3-[(3-méthyl-5-hydroxy-1-phényl-1H-pyrazol-4-yl)azo]-triméthyl-ammonio-benzène, le chlorure de 8-[(4-amino-phényl)azo]-7-hydroxy-N,N,N-triméthyl-2-naphthalène-aminium, le chlorure de 8-[(4-amino-3-nitrophényl)azo]-7-hydroxy-N,N,N-triméthyl-2-naphthalène-aminium, le chlorure de 8-[(4-amino-2-nitrophényl)-azo]-7-hydroxy-N,N,N-triméthyl-2-naphthalène-aminium, le chlorure de 7-hydroxy-N,N,N-triméthyl-8-{[2-(méthyloxy)phényl]-azo}-2-naphthalène-aminium, le chlorure de 3-((4-amino-6-bromo-5,8-dihydro-1-hydroxy-8-imino-5-oxo-2-naphthalényl)amino)-N,N,N-triméthyl-benzène-aminium et le bromure de N,N-diméthyl-3-{[4-(méthylamino)-9,10-dioxo-9,10-dihydro-1-anthracényl]-amino}-N-propyl-1-propanaminium.

8. Composition selon la revendication 6 ou 7, **caractérisée en ce qu'**elle contient les autres colorants en une quantité totale de 0,01 à 15 % en poids.

9. Composition selon l'une quelconque des revendications 4 à 8, **caractérisée en ce qu'**elle contient au moins un polymère naturel ou synthétique ou un polymère d'origine naturelle, modifié, et se trouve sous forme d'un fixateur de nuance ou d'un fixateur de couleur.

10. Composition selon l'une quelconque des revendications 4 à 9, **caractérisée en ce qu'**elle est une composition de teinture pour cheveux.
